(19) Europäisches Patentamt
European Patent Office
Office européen des brevets

(11) Publication number: **0 125 803**
**A2**

(12) # EUROPEAN PATENT APPLICATION

(21) Application number: 84302566.9

(22) Date of filing: 16.04.84

(51) Int. Cl.³: **C 07 D 211/90**
C 07 D 211/78, C 07 D 401/04
C 07 D 405/04, C 07 D 413/04
C 07 D 401/06, A 61 K 31/445

(30) Priority: 27.04.83 GB 8311519
27.04.83 GB 8311520
27.04.83 GB 8311521
01.10.83 GB 8326362
15.10.83 GB 8327660
15.10.83 GB 8327661
18.11.83 GB 8330852
22.12.83 GB 8334285
22.12.83 GB 8334286
22.12.83 GB 8334287

(43) Date of publication of application:
21.11.84 Bulletin 84/47

(84) Designated Contracting States:
AT BE CH DE FR GB IT LI LU NL SE

(71) Applicant: FISONS plc
Fison House Princes Street
Ipswich Suffolk IP1 1QH(GB)

(72) Inventor: Baxter, Andrew John Gilby
78 Mount Pleasant
Keyworth Nottingham(GB)

(72) Inventor: Dixon, John
35 de Verdun Avenue
Belton Leicestershire(GB)

(72) Inventor: Gould, Kenneth John
3A Turvey Lane
Long Whatton Leicestershire(GB)

(72) Inventor: McInally, Thomas
24 Borrowell
Kegworth Leicestershire(GB)

(72) Inventor: Tinker, Alan Charles
97 Knightthorpe Road
Loughborough Leicestershire(GB)

(74) Representative: Craig, Christopher Bradberry et al,
Fisons plc 12 Derby Road
Loughborough Leicestershire LE11 0BB(GB)

(54) **Pharmaceutically active dihydropyridines.**

(57) There are described compounds of formula I,

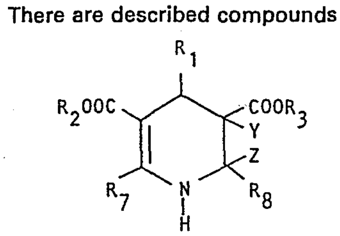

I

in which $R_1$ represents benzofurazanyl, pyridyl or phenyl, the pyridyl or phenyl being substituted,

$-COOR_2$ and $-COOR_3$ are various ester groups,

Y and Z together form a bond, and additionally, when $R_8$ is an electron withdrawing group Y may be hydrogen and Z may be hydroxy,

one of $R_7$ and $R_8$ represents alkyl C1 to 6 and the other represents $-CONR_{10}R_{11}$; $-CSNH_2$; $-C(=NH)SR_9$; $-S(O)_mR_9$; phenyl optionally substituted by one or more of alkyl C1 to 6, halogen, alkoxy C1 to 6 or nitro; alkyl C1 to 6 substituted by halogen; or furanyl,

or $R_7$ and $R_8$ may be the same or different and each represents phenyl optionally substituted by one or more of alkyl C1 to 6, halogen, alkoxy C1 to 6 or nitro; amino; alkyl C1 to 6 substituted by halogen; $-CN$; $-CH_2OH$; $-CHO$ or $-CH(OR_9)_2$,

m is 0 or 1

$R_9$ is alkyl C1 to 6, and

$R_{10}$ and $R_{11}$ each independently represent hydrogen or alkyl C1 to 6, or together with the nitrogen atom to which they are attached form a 5 or 6 membered heterocyclic ring.

There are also described processes for making the compounds, and pharmaceutical, eg calcium antagonist, formulations containing them.

EP 0 125 803 A2

Croydon Printing Company Ltd.

PHARMACEUTICALLY ACTIVE DIHYDROPYRIDINES

This invention relates to new compounds, methods for their preparation and compositions containing them.

A wide variety of dihydropyridines have been described as being useful as pharmaceuticals and some, notably nifedipine, have been sold for this use.

We have now found a new group of pyridine derivatives which have pharmacological activity.

According to the invention we provide compounds of formula I,

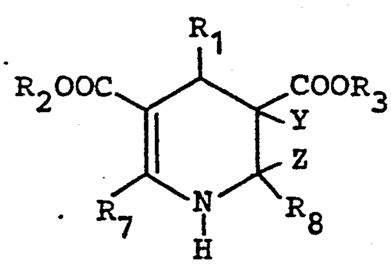

I

in which $R_1$ represents benzofurazanyl, pyridyl or phenyl, the pyridyl or phenyl being substituted by one or more of the groups halogen, nitro, -CN, $-OR_9$, $-S(O)_pR_9$, or alkyl C1 to 6 optionally substituted by halogen,

p is 0, 1 or 2,

$R_2$ and $R_3$, which may be the same or different, each represent hydrogen; alkyl C1 to 6 optionally substituted by one or more of the groups halogen, cyano, $-XR_4$, $-NR_5R_6$ or phenyl; cycloalkyl C3 to 8

optionally substituted by alkyl C1 to 6; a 4, 5 or 6 membered oxygen or nitrogen containing heterocyclic ring which is optionally substituted by alkyl C1 to 6 the alkyl in turn optionally being substituted by one or more phenyl groups;

$R_5$ and $R_6$, which may be the same or different, each represent alkyl C1 to 6 optionally substituted by phenyl,

Y and Z together form a bond, and additionally, when $R_8$ is an electron withdrawing group Y may be hydrogen and Z may be hydroxy,

one of $R_7$ and $R_8$ represents alkyl C1 to 6 and the other represents $-CONR_{10}R_{11}$; $-CSNH_2$; $-C(=NH)SR_9$; $-S(O)_mR_9$; phenyl optionally substituted by one or more of alkyl C1 to 6, halogen, alkoxy C1 to 6 or nitro; alkyl C1 to 6 substituted by halogen; or furanyl,

or $R_7$ and $R_8$ may be the same or different and each represents phenyl optionally substituted by one or more of alkyl C1 to 6, halogen, alkoxy C1 to 6 or nitro; amino; alkyl C1 to 6 substituted by halogen; -CN; $-CH_2OH$; -CHO or $-CH(OR_9)_2$,

X is O or S,

m is 0 or 1,

$R_4$ is alkyl C1 to 6 or phenyl,

$R_9$ is alkyl C1 to 6,

$R_{10}$ and $R_{11}$ each independently represent hydrogen or alkyl C1 to 6, or together with the nitrogen atom to which they are attached form a 5 or 6 membered heterocyclic ring,

provided that A) when $R_7$ is alkyl C1 to 6, Y and Z together form a bond, and

i) $R_1$ represents benzofurazanyl then $R_8$ does not represent $-CF_3$, or

ii) when $R_1$ represents 2-nitrophenyl, or 2-chlorophenyl and $R_2$ and $R_3$ are both ethyl, then $R_8$ does not represent mono-chloromethyl, or

iii) when $R_1$ represents 3-nitrophenyl and $R_2$ and $R_3$ are both ethyl, then $R_8$ does not represent unsubstituted phenyl,

B) when neither of $R_7$ and $R_8$ is alkyl C1 to 6, Y and Z together form a bond and

iv) $R_2$ and $R_3$ are both ethyl then $R_7$ and $R_8$ are not both $-CF_3$, or

v) one of $R_7$ or $R_8$ is amino then the other is not phenyl or amino, or

vi) one of $R_7$ or $R_8$ is $-CN$, $-CH_2OH$, $-CHO$ or $-CH(OR_9)_2$ then the other is not $-CN$, $-CH_2OH$, $-CHO$ or $-CH(OR_9)_2$, and

C) both of $R_7$ and $R_8$ are not optionally substituted phenyl,

and pharmaceutically acceptable acid addition salts of those compounds containing a basic nitrogen atom.

According to the invention we also provide the compounds of formula I without proviso ii) for use as pharmaceuticals.

According to the invention we further provide a process for the production of a compound of formula I, or a pharmaceutically acceptable acid addition salt thereof, which comprises

a) reaction of a compound of formula II,

$R_1CHO$ II

with compounds of formulae III and IV,

$R_2OOCCH=C(R_7)NH_2$ III

$R_3OOCCH_2COR_8$ IV

in which formulae $R_1$, $R_2$, $R_3$, $R_7$ and $R_8$ are as defined above,

b) reaction of a compound of formula V,

$R_1CH=C(COOR_3)COR_8$ V

in which $R_1$, $R_3$ and $R_8$ are as defined above, with a compound of formula III,

c) production of a compound of formula I in which Y and Z together form a bond by dehydration of a compound of formula VII,

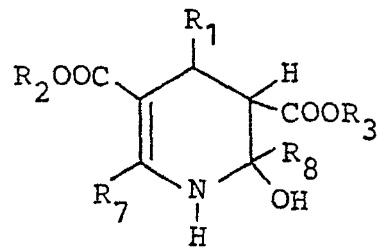

VII

in which $R_1$, $R_2$, $R_3$, $R_7$ and $R_8$ are as defined above,

d) production of a compound of formula I in which m is 1 or p is 1 or 2 by selective oxidation of a corresponding compound of formula I in which m is 0, or p is 0 or 1 respectively,

e) production of a compound of formula I in which one of $R_7$ and $R_8$ is $-CONR_{10}R_{11}$ by reaction of an acid halide, imidazole or a mixed anhydride of a corresponding compound of formula I in which one of $R_7$ and $R_8$ is -COOH with a compound $HNR_{10}R_{11}$ in which $R_{10}$ and $R_{11}$ are as defined above, or, when the group $-NR_{10}R_{11}$ in the product represents an imidazole, reacting the free carboxylic acid of formula I with N,N'-carbonyldiimidazole,

f) production of a compound of formula I in which one of $R_7$ and $R_8$ is $-CSNH_2$ by reaction of a corresponding compound of formula I in which one of $R_7$ and $R_8$ is -CN with hydrogen sulphide,

g) isomerising a 3,4-dihydropyridine to a corresponding compound of formula I,

h) production of a compound of formula I in which one of $R_7$ and $R_8$ is $-C(=NH)SR_9$ by reaction of a corresponding compound of formula I in which one of $R_7$ and $R_8$ is $-CSNH_2$ with a compound $R_9$-hal, in which $R_9$ is as defined above and hal is a halogen atom,

i) reaction of a compound of formula IV with ammonia and a compound of formula VI,

$R_1CH=C(COOR_2)COR_7$ VI

or reaction of a compound of formula V with ammonia and a compound of formula VII,

$R_2OOCCH_2COR_7$ VII

or reaction of compounds of formulae II, IV and VII with ammonia,

in which formulae $R_1$, $R_2$, $R_3$, $R_7$ and $R_8$ are as defined above,

j) production of a compound of formula I in which Y and Z together form a bond and one or both of $R_7$ and $R_8$ is $-CHF_2$ or $-CH_2F$ by reaction of a corresponding compound of formula I in which Y and Z together form a bond and one or both of $R_7$ and $R_8$ is -CHO or $-CH_2L$, where L is -OH or a good leaving group, respectively with a fluorinating agent,

k) production of a compound of formula I in which one of $R_7$ and $R_8$ is -CHO by selective hydrolysis of a corresponding compound of formula I in which one of $R_7$

and $R_8$ is $-CH(OR_9)_2$,

l) production of a compound of formula I in which one of $R_7$ and $R_8$ is $-CH_2OH$ by selective reduction of a corresponding compound of formula I in which one of $R_7$ and $R_8$ is -CHO,

m) production of a compound of formula I in which one of $R_7$ and $R_8$ is -CN by elimination of ROH from a corresponding compound of formula I in which one of $R_7$ and $R_8$ is -CH=NOR, and -OR is a good leaving group,

n) production of a compound of formula I in which at least one of $R_2$ and $R_3$ is hydrogen by reductive cleavage or hydrolysis of a corresponding compound of formula I in which at least one of $R_2$ and $R_3$ is other than hydrogen,

o) production of a compound of formula I in which at least one of $R_2$ and $R_3$ is other than hydrogen by esterification or transesterification of a corresponding compound of formula I in which at least one of $R_2$ and $R_3$ is hydrogen or is a group $R_2$ or $R_3$ other than that desired in the end product, or

p) production of an optical isomer of a compound of formula I by resolution of a mixture of optical isomers of the compound,

and where desired or necessary converting the resulting compound of formula I to a pharmaceutically

acceptable acid addition salt thereof or vice versa.

The reaction of process a) may be carried out by subjecting the compounds of formulae II, III and IV to an elevated temperature, eg of from about 20° to 140°C in the presence or absence of a suitable solvent, eg a lower alkanol.

Processes b) and i) may be carried out under similar conditions to process a). In processes a), b) and i) when Y and Z in the final product are together to form a bond dehydration is generally required as a separate process step when $R_8$ is an electron withdrawing group, eg $-CF_3$, perhaloalkyl-, nitro- or mono- or di-chlorophenyl or unsubstituted phenyl. The presence of a base, eg diethylamine or ammonia, tends to inhibit dehydration in these processes. We prefer not to use process a), b) or i) when $R_7$ or $R_8$ is $-C(=NH)SR_9$, -CN, $-CH_2OH$ or -CHO, or when $R_2$ or $R_3$ is hydrogen.

Process c) may be carried out in a solvent which is inert under the reaction conditions, eg methylene chloride, and in the presence of a dehydrating agent, eg trifluoracetic anhydride, and a base, eg pyridine. The dehydration may also be effected using diethylaminosulphur trifluoride. The reaction may be carried out at from about 0° to 40°C. The compounds of formula VII may be formed as intermediates, which may

or may not be isolated, in processes a), b) and i). Under certain circumstances, eg when $R_8$ is not an electron withdrawing group, the compound of formula VII may dehydrate spontaneously to yield the compound of formula I in which Y and Z together form a bond. When diethylaminosulphur trifluoride is used in this process and $R_8$ is $CH_2OH$ or CHO in the starting material the $-CH_2OH$ or -CHO will, as in process j), be converted to $-CH_2F$ and $-CHF_2$ respectively.

Process d) may be carried out using a suitable oxidising agent, eg peracetic acid. The reaction may be carried out in a suitable solvent, eg a mixture of methanol and acetic acid. We prefer not to use this process when $R_7$ or $R_8$ is $-C(=NH)SR_9$.

Process e) may be carried out by treating the acid halide, imidazole or the mixed anhydride (which compounds may be prepared by conventional processes known per se), with aqueous ammonia or the amine $HNR_{10}R_{11}$ at a temperature of from $0^o$ to $30^oC$. When Z is -OH, or $R_7$ or $R_8$ is $-CH_2OH$, we prefer to use the imidazole or a mixed anhydride. We prefer not to use this process when $R_2$ or $R_3$ is hydrogen.

Process f) may be carried out by treating the nitrile starting material with hydrogen sulphide gas in a suitable solvent, eg pyridine. The reaction is preferably carried

out in the presence of a base, eg triethylamine, and may conveniently be carried out at a temperature of from 10 to 30°C.

Process g) may be carried out under basic conditions, eg in the presence of an alkylamine such as triethylamine. This process is particularly applicable when $R_8$ is both electron withdrawing and bulky.

Process h) may be carried out in a solvent which is inert under the reaction conditions, eg diethyl ether. We prefer hal to be iodine.

Process j) is preferably carried out at a temperature of from about -70° to 100°C, and in a solvent which is inert under the reaction conditions, e.g. a halogenated hydrocarbon and preferably methylene chloride. The fluorinating agent is preferably a dialkylaminosulphur trifluoride, e.g. diethylaminosulphur trifluoride, or (2-chloro-1,1,2-trifluoroethyl)diethylamine. The group L may be, for example, $-OSO_2Rx$, where Rx is alkyl C1 to 6, e.g. methyl, or aryl, e.g. p-tolyl.

The hydrolysis of process k) may be carried out using an aqueous acid, for example hydrochloric acid (eg 0.5 to 2.5 molar) in a water miscible organic solvent, eg acetone or tetrahydrofuran. The reaction may be carried out at a temperature of from about -10 to 50°C.

The reduction of process l) may be carried out either

chemically or catalytically, eg by use of sodium borohydride in an alcoholic solvent, eg methanol or ethanol, at a temperature of from about 0 to 50°C.

The elimination of process m) may be carried out using a variety of dehydrating agents which will not adversely effect the other substituents in the molecule, e.g., an excess of acetic anhydride, thionyl chloride in ether or N,N'-dicylohexylcarbodiimide in pyridine. The group -OR may be, for example, a 2,4-dinitrophenoxy group. The reaction may be carried out at a temperature of from about 0° to 150°C depending on the reagent and solvent used. The oxime may, if desired, be formed *in situ* from the corresponding formyl compound using conventional methods known *per se*.

The reductive cleavage of process n) may be carried out chemically, eg using zinc and formic acid. The reaction may conveniently be carried out in a solvent which is inert under the reaction conditions, eg acetonitrile. When process n) involves a hydrolysis the hydrolysis may be carried out using conventional techniques known *per se*.

Process o) may, when it involves an esterfication, be carried out using the appropriate alcohol, preferably in excess and in the presence of a dehydrating agent, eg dicyclohexylcarbodiimide, or under similar conditions to

process e). The reaction may conveniently be carried out in a solvent which is inert under the reaction conditions, eg ethyl acetate. When a transesterification is involved the process may be carried out by treating the starting ester with the sodium alcoholate corresponding to the desired ester moiety.

The resolution of process p) may be carried out by means of conversion of the mixture to, when $R_2$ or $R_3$ is H, a salt with an optically active base or an ester with an optically active alcohol (eg $CCl_3(C_6H_5)CHOH$ or $C_6H_5(OCH_3)CHCH_2OH$), or, when $R_2$ or $R_3$ is aminoalkyl, a salt with an optically active acid and separation of the product by selective crystallisation, or, preferably, by means of high performance liquid chromatography (HPLC). The separated product may then be converted to the desired optically active acid or ester by, for example, process n) or o).

The starting materials for the above processes are either known, or if they are not specifically known they may be made by processes described in the Examples, or they may be made from known compounds using one or more process steps which are known *per se* or are analogous to those described in the Examples.

Certain of the compounds of formula II are novel and the invention therefore also provides those compounds of

formula II in which $R_1$ is 2-chloro-3-trifluoromethyl phenyl or phenyl substituted by three substituents selected from chloro-, fluoro- and $-CF_3$. Specifically we provide 2,3-dichloro-6-fluorobenzaldehyde, 3-chloro-6-fluoro-2-(trifluoromethyl)benzaldehyde and 2-chloro-3-(trifluoromethyl)benzaldehyde.

The compounds of formula I and the intermediates therefor may be isolated from their reaction mixtures using conventional processes, eg crystallisation or chromatography.

The compounds of formula I, and the pharmaceutically acceptable salts thereof, are useful because they exhibit pharmacological properties in animals. More particularly they block the entry of calcium into vascular and cardiac muscle leading to falls in blood pressure, inotropy and heart rate. They are active in the following systems:-

(a) Relaxation of contracted vascular smooth muscle. Van Breemen, Aaronson, Loutzenhiser and Meisheri, Chest, 78, Supplement, 157-165, 1980.

(b) Reduction of inotropy and chronotropy of isolated atria. Henry, Excerpta Med. Int. Congr. Ser., 474, 14-23, 1979.

(c) Reduction of blood pressure and increase cardiac output in anaesthetised dogs. Hirakawa, Ito, Kondo, Watanbe, Hiei, Banno & Hyase, Arzneim-Forsch, 22,

344-349, 1972.

(d) Reduction of blood pressure in conscious dogs when given by the intravenous and oral routes. Newman, Bishop, Peterson, Leroux & Horowitz, J Pharm. Exp. Ther. 201, 723-730, 1977.

The compounds are thus indicated for use in the treatment of renovascular, malignant or essential hypertension (including hypertensive emergencies), pulmonary hypertension, vasospastic angina, chronic stable angina and congestive heart failure. Other indications are the treatment of renal failure, cardiac arrhythmias, hypertrophic cardiomyopathy, cerebrovascular diseases (including cerebral haemorrhage, ischaemia and vasospasm, migraine, altitude sickness and hearing loss), peripheral vascular diseases (including Raynauds syndrome, intermittent claudication and digital ulceration); use as a cardioplegic agent during surgery eg in cardiopulmonary bypass, and for the treatment of, and protection against, myocardial infarction and ischaemia.

By virtue of their ability to inhibit calcium entry into other cells and tissues the compounds are also indicated in the treatment of thrombosis, atherosclerosis, respiratory diseases (including asthma and bronchitis) glaucoma, aldosteronism, uterine hypermotility and for the relief of oesophageal and skeletal muscle spasm.

For the above uses the dosage will depend upon the compound used, the route of administration and the effect desired, but in general will be in the range of 0.1-10mg per kilogram body weight per day. For man the indicated total daily dose will be from about 5-500mg, preferably from 5 to 200mg and more preferably from 5 to 100mg, which may be administered preferably once daily, or in divided doses of about 1-200mg, preferably 2 to 25mg, e.g. 2 to 4 times per day.

The compounds of formula I are advantageous in that they possess greater potency (e.g. with respect to hypotensive and direct negative chronotropic effects), produce a lower level of reflex tachycardia, are more selective (e.g. for vascular smooth muscle vs cardiac muscle), produce less depression of cardiac contractility, are longer acting, are more readily absorbed or less readily metabolised, are more easily formulated, possess less, or less undesirable, side effects, are more stable or have other more beneficial properties than known compounds of similar structure.

The compounds of the invention may be administered by a wide variety of routes and may act systemically or locally. Thus the compounds may be administered by oral or nasal inhalation to the lung, to the buccal cavity, oesophageally, rectally, topically to the skin, the eye or

to other available surfaces of the body; by injection, eg intravenously, intramuscularly, intraperitoneally, or by surgical implant.

When $R_2$ and/or $R_3$ represents a 4, 5 or 6 membered oxygen or nitrogen containing heterocyclic ring that ring may be an oxetanyl, azetidinyl, piperidinyl or tetrahydropyranyl ring. $R_2$ and/or $R_3$ may also represent $-(CH_2)_nXR_4$, $-(CH_2)_nCN$, $-CH(C_6H_5)CCl_3$ or $-(CH_2)_nR_5R_6$ in which n is 4, 3 or preferably 2.

When $R_{10}$ and $R_{11}$ together with the nitrogen atom to which they are attached form a 5 or 6 membered heterocyclic ring we prefer that ring to be a morpholino or imidazolyl ring.

We prefer compounds of formula I in which Y and Z together form a bond. We also prefer those compounds in which one of $R_7$ and $R_8$ is alkyl C1 to 6, eg methyl. We further prefer those compounds in which one of $R_7$ and $R_8$ is mono-, di- or tri- fluoromethyl. We particularly prefer one of $R_7$ and $R_8$ to be mono- fluoromethyl.

Groups $R_8$ which are electron withdrawing include alkyl C1 to 6 substituted by 2 or more halogen atoms; furanyl and phenyl optionally substituted by one or more of alkyl C1 to 6, halogen, alkoxy C1 to 6 or nitro.

Preferred electron withdrawing significances of $R_8$ are $-CCl_3$, $-CF_3$, $-CF_2CF_3$, phenyl, 4-nitrophenyl, 3,4-dichlorophenyl, 4-chlorophenyl and 3-chlorophenyl.

Values for $R_1$ include nitrophenyl; (trifluoromethyl)phenyl; mono- or poly-fluorophenyl; mono- or poly-chlorophenyl; chloro- and/or fluoro-(trifluoromethyl)phenyl; (alkylthio)pyridyl; alkyl- and/or chloro- and/or alkoxy-nitrophenyl; mixed chloro- and fluoro-phenyl; mono- or poly- alkoxy-phenyl; alkylphenyl; (alkylthio)phenyl; (alkylsulphonyl)phenyl; and 4-benzofurazanyl. Values for $R_2$ and $R_3$ are alkyl C1 to 4, 2-alkoxy C1 to 3 - ethyl, 2-phenoxy- ethyl, cycloalkyl C4 to 6 optionally substituted by methyl, the saturated 4, 5 or 6 membered heterocyclic groups as defined immediately above and optionally substituted by phenylmethyl or diphenylmethyl, alkyl C1 to 4 - (phenylmethyl)aminoethyl, cyano- or alkyl C1 to 4 - thio-alkyl C1 to 4; phenyl alkyl C1 to 4 or $-CH(C_6H_5)CCl_3$. Values of $R_8$ are chloro- or fluoro- alkyl C1 or 2, $-CSNH_2$, $-CON(\text{alkyl C 1 to 4})_2$, -COmorpholino, -COimidazolyl, -C(=NH)S-alkyl C1 to 4, -S-alkyl C1 to 4, -S(O)-alkyl C1 to 4, or phenyl substituted by one or two chlorine, nitro, methoxy or methyl groups, e.g. in the 4- and/or 3- positions. $R_7$ may be methyl. When $R_7$ is not alkyl $R_7$ and $R_8$ are

preferably selected from a fluoromethyl group, e.g. $-CH_2F$, $-CHF_2$ or $-CF_3$; $-CHO$; $-CH(OC_2H_5)_2$; phenyl and $-CH_2OH$. The Examples illustrate various permutations of substituents. The individual substituents exemplified may also be permutated in other combinations.

As a preferred group of compounds of formula I we provide those in which $R_1$ is phenyl carrying a 2-nitro or a 2-$CF_3$ group or at least two substituents selected from chloro; fluoro; alkyl C1 to 6, eg methyl; $-CF_3$ and nitro; $R_2$ is alkyl C1 to 6, eg isopropyl, cyclopentyl or cyclobutyl or is oxetan-3-yl; $R_3$ is alkyl C1 to 6, eg methyl; $R_7$ is alkyl C1 to 6, eg methyl; $R_8$ is fluoromethyl, eg mono-, di- or tri-fluoromethyl; and Y and Z together form a bond.

As a most preferred group of compounds of formula I we provide those in which $R_1$ is phenyl carrying at least two substituents selected from chloro, fluoro, $-CF_3$, methyl and nitro, $R_3$ and $R_7$ are both methyl, $R_8$ is $-CH_2F$, $R_2$ is isopropyl or cyclopentyl and Y and Z together form a bond.

A specific group of compounds of formula I are those in which $R_1$ represents benzofurazanyl, pyridyl or phenyl, the pyridyl or phenyl being substituted by one or more of the groups halogen, nitro, trihalomethyl or $-SR_9$; $R_2$ and $R_3$ each represent alkyl C1 to 6,

$-(CH_2)_n R_4$, $-(CH_2)_nCN$, $-CH(C_6H_5)CCl_3$ or $-(CH_2)_n NR_5R_6$; Y and Z together form a bond; one of $R_7$ and $R_8$ represents alkyl C1 to 6 and the other represents $-CONR_{10}R_{11}$; $-CSNH_2$; $-C(=NH)SR_9$; $-S(O)_mR_9$; phenyl substituted by one or more of alkyl C1 to 6, halogen, alkoxy C1 to 6 or nitro; or alkyl C1 to 6 substituted by halogen; $R_4$ and $R_9$ are each alkyl C1 to 6; and $R_{10}$ and $R_{11}$ each represent hydrogen or alkyl C1 to 6, and provisos i) and ii) apply.

According to our invention we also provide a pharmaceutical composition comprising preferably less than 80%, more preferably less than 50%, eg 1 to 20%, by weight of a compound of formula I, or a pharmaceutically acceptable salt thereof, in admixture with a pharmaceutically acceptable adjuvant, diluent or carrier.

Thus the compound may be put up as a tablet, capsule, dragee, suppository, suspension, solution, injection, implant, a topical, eg transdermal, preparation such as a gel, cream, ointment, aerosol or a polymer system, or an inhalation form, e.g. an aerosol or a powder formulation.

We prefer compositions which are designed to be taken oesophageally and to release their contents in the gastrointestinal tract. Thus we prefer tablets which may, for example, be made by direct compression. In such a process the active ingredient is mixed with one or more of

modified forms of starch, calcium phosphate, a sugar eg lactose, microcrystalline cellulose and/or other directly compressible excipients, together with lubricant(s), eg stearic acid or magnesium stearate, flow aid(s), eg talc or colloidal silicon dioxide, and disintegrant(s), eg starch, substituted sodium carboxymethyl cellulose, cross linked sodium carboxy methyl cellulose, carboxy methyl starch and cross linked polyvinylpyrrolidone. Tablets are then formed by direct compression, and may be sugar or film coated e.g. with hydroxypropylmethylcellulose.

Alternatively the active ingredient may be granulated before tabletting. In such cases the active ingredient is mixed with one or more of starch, calcium phosphate, a sugar eg lactose, microcrystalline cellulose or other suitable excipients and granulated with a binder such as starch, pregelled starch, polyvinylpyrrolidone, gelatine, a modified gelatine, or a cellulose derivative, eg hydroxypropylmethylcellulose. The mass is then dried, sieved and mixed with lubricant(s), flow aid(s) and disintegrant(s), such as described in the previous paragraph. Tablets are then formed by compression of the granules, and may be sugar or film coated, eg with hydroxypropylmethylcellulose.

As a further alternative a powder, blend or granules, such as are described above as intermediates in

tabletting, may be filled into a suitable, eg gelatine, capsule.

In order to improve the bioavailability, or decrease variability of availability, of the active ingredient the compound may be:-

a) dissolved in a suitable solvent, eg polyethylene glycol, Gelucaire, arachis oil, a (hydrogenated) vegetable oil or beeswax and the solution is then filled into a gelatine capsule,

b) produced as a spray-dried or freeze-dried form prior to mixing with other excipients,

c) milled and/or micronised to produce a powder with a large surface area prior to mixing with other excipients,

d) made into a solution and distributed over an inert excipient having a large surface area, eg colloidal silicon dioxide. The solvent is evaporated and further excipients added,

e) formed into a complex with cyclodextrin prior to mixing with other excipients. This complex also assists in increasing light stability, or

f) made into a solid solution or co-precipitated, eg with polyvinylpyrrolidone, polyethyleneglycol, modified cellulose, hydroxypropylmethylcellulose, urea or a sugar prior to mixing with further excipients.

The compounds, either in their normal form or in a

modified form, eg as described immediately above, may be formulated in a controlled release form. Thus the compound may be dispersed, or contained in, a polymer matrix formed from, for example, ethylcellulose, hydroxypropylmethylcellulose or an acrylate/methacrylate polymer. Alternatively the compound may be formulated as a tablet or beads which are surrounded by a semi-permeable membrane, eg shellac, ethylcellulose or an acrylate/methacrylate polymer.

The compounds of this invention may be given in combination with other pharmaceutically active compounds, eg diuretics, beta-blockers, antihypertensives or inotropic agents. The dosage of the other pharmaceutically active compound can be that conventionally used when the compound is administered on its own, but is preferably somewhat lower. To illustrate these combinations, a compound of this invention effective in the range, eg 5-100 milligrams per day, can be combined at levels ranging, eg from 1-200 milligrams per day with the following beta-blockers, antihypertensives and diuretics in dose ranges per day as indicated:

hydrochlorothiazide (15-200mg), chlorothiazide (125-2000mg), ethacrynic acid (15-100mg), amiloride (5-20mg), furosemide (5-80mg), propanolol (20-480mg), timolol (5-50mg), captopril (10-500mg), methyldopa

(65-2000mg) or digoxin (0.1-0.5mg). In addition, the triple drug combinations of hydrochlorothiazide (15-200mg) plus amiloride (5-20mg) plus a compound of this invention (3-200mg) and hydrochlorothiazide (15-200mg) plus timolol (5-50mg) plus a compound of this invention (3-200mg), are provided. The above dose ranges may be adjusted on a unit basis as necessary to permit divided daily dosage. Also, the dose may vary depending on the severity of the disease, weight of patient and other factors which a person skilled in the art will recognise.

Certain of the compounds of formula I are assymetric and exhibit optical isomerism. Such compounds may be separated into their optical isomers using process p) or may be made by stereospecific syntheses using conventional techniques know *per se*.

The invention therefore provides the compounds as their individual optical isomers (we prefer the (+) isomers), and racemic or other mixtures of the individual isomers.

In those compounds in which Y is hydrogen and Z is -OH there will be at least 3 assymetric carbon atoms and the corresponding number of isomers is provided.

Certain of the compounds of the invention can form solvates, eg hydrates or alcoholates, and certain of the compounds are light sensitive and should therefore be

produced, handled, stored and formulated in such a manner that they are not subjected to degrading amounts of light of the appropriate wavelengths.

The invention is illustrated, but in no way limited by the following Examples in which temperatures are in degress centigrade.

Example 1

3-(Methyl) 5-(1-methylethyl) 4-(2,3-dichlorophenyl)-2--(fluoromethyl)-1,4-dihydro-6-methyl-3,5-pyridinedicarboxylate

2,3-Dichlorobenzaldehyde (1.75g, 10mmoles), methyl 4-fluoro-3-oxobutanoate (1.34g, 10mmoles) and 1-methylethyl 3-amino-2-butenoate (1.43g, 10mmoles) were heated with stirring at 90$^{o}$ for 2.5 hours. The reaction mixture was dissolved in ethyl acetate and chromatographed on silica eluting with petroleum ether (60-80$^{o}$)/ethyl acetate mixtures. Pure fractions were combined and evaporated to dryness. The title compound (1.6g) was obtained by crystallisation from 2-propanol. mp 148-9$^{o}$.

Example 2

3-Methyl 5-(1-methylethyl) 2-(fluoromethyl)-1,4-dihydro-6-methyl-4-(2-methyl-3-nitrophenyl)-3,5-pyridinedicarboxylate

2-Methyl-3-nitrobenzaldehyde (1.23g, 7.5mmoles), methyl 4-fluoro-3-oxobutanoate (1.0g, 7.5mmoles) and 1-methylethyl 3-amino-2-butenoate (1.07g, 7.5mmoles) were heated with stirring at 80$^{o}$ for 2.5 hours. The cooled residue was chromatographed twice on silica eluting first with ethyl acetate/petroleum ether (60-80$^{o}$) and then with ethyl acetate/methylene chloride. The title compound (0.61g) was obtained by crystallisation from a mixture of

petroleum ether (60-80°) and 2-propanol mp 132-133°.

Example 3

5-Cyclopentyl 3-methyl 4-(2,3-dichlorophenyl)-2-(fluoromethyl)-1,4-dihydro-6-methyl-3,5-pyridinedicarboxylate

2,3-Dichlorobenzaldehyde (1.31g, 7.5mmoles), methyl 4-fluoro-3-oxobutanoate (1.0g, 7.5mmoles) and cyclopentyl 3-amino-2-butenoate (1.26g, 7.5mmoles) were heated at 90° with stirring under nitrogen for 2.5 hours. The reaction mixture was dissolved in ethyl acetate, dried ($MgSO_4$) and the solvent was removed *in vacuo*. The residue was chromatographed on silica eluting with ethyl acetate/methylene chloride mixtures. The title compound (0.95g) was obtained after crystallisation from petroleum ether (60-80°) mp 148-50°.

Example 4

3-Methyl 5-(1-methylethyl) 4-(3-chloro-6-fluoro-2-(trifluoromethyl)phenyl)-2-(fluoromethyl)-1,4-dihydro-6-methyl-3,5-pyridinedicarboxylate

3-Chloro-6-fluoro-2-(trifluoromethyl)benzaldehyde (1.3g, 5.7mmoles), methyl 4-fluoro-3-oxobutanoate (0.77g, 5.7mmoles) and 1-methylethyl 3-amino-2-butenoate (0.82g, 5.7mmoles) were heated under nitrogen with stirring for 1.5 hours at 90°, followed by 1.5 hours at 100° and then 1 hour at 110°. The cooled reaction mixture was

chromatographed twice on silica first using methylene chloride as eluent and then toluene/ethyl acetate mixtures. The title compound (0.2g) was obtained after crystallisation from petroleum ether (60-80°) mp 142-3°.

Example 5

3-Methyl 5-(1-methylethyl) 4-(2-chloro-3-nitrophenyl)-2-(fluoromethyl)-1,4-dihydro-6-methyl-3,5-pyridinedicarboxylate

2-Chloro-3-nitrobenzaldehyde (1.38g, 7.5mmoles), methyl 4-fluoro-3-oxobutanoate (1.0g, 7.5mmoles) and 1-methylethyl 3-amino-2-butenoate (1.06g, 7.5mmoles) were heated at 90° for 2.5 hours. The reaction mixture was chromatographed on silica eluting with petroleum ether (60-80°)/ethyl acetate mixtures. The title compound (1.35g) was obtained after crystallisation from petroleum ether (60-80°)/2-propanol. mp 156-7°.

Example 6

3-Methyl 5-(1-methylethyl) 2-(fluoromethyl)-4-(2-fluoro-6-(trifluoromethyl)phenyl)-1,4-dihydro-6-methyl-3,5-pyridinedicarboxylate

2-Fluoro-6-(trifluoromethyl)benzaldehyde (1.51g, 7.8mmoles), methyl 4-fluoro-3-oxobutanoate (1.06g, 7.8mmoles) and 1-methylethyl 3-amino-2-butenoate (1.13g, 7.8mmoles) were heated at 90° under nitrogen with

stirring for 2 hours. The cooled reaction mixture was chromatographed twice; first eluting with toluene/ethyl acetate mixtures and then with ethyl acetate/petroleum ether (60-80°) mixtures. The title compound (0.1g) was obtained on evaporation of the pure fractions mp 82-4°.

Example 7

3-Methyl 5-(1-methylethyl) 4-(2,3-dichloro-6-fluorophenyl)-2-(fluoromethyl)-1,4-dihydro-6-methyl-3,5-pyridinedicarboxylate

2,3-Dichloro-6-fluorobenzaldehyde (1.44g, 7.5mmoles), methyl 4-fluoro-3-oxobutanoate (1.0g, 7.5mmoles) and 1-methylethyl 3-amino-2-butenoate (1.06g, 7.5mmoles) were heated at 90° under nitrogen with stirring for 2.5 hours. The cooled reaction mixture was chromatographed on silica eluting with ethyl acetate/methylene chloride mixtures. The title compound (1.1g) was obtained by crystallisation from a petroleum ether (60-80°)/2-propanol mixture mp 129-31°.

The compounds of Examples 8 to 49 were prepared using appropriate starting materials and the method described in Examples 1-7.

Example 8

5-Ethyl 3-methyl 2-(fluoromethyl)-1,4-dihydro-6-methyl-4-(3-nitrophenyl)-3,5-pyridinedicarboxylate

Recrystallised from 2-propanol. mp 127-9°.

Example 9

3-Methyl 5-(1-methylethyl) 2-(fluoromethyl)-1,4-dihydro-6-methyl-4-(3-nitrophenyl)-3,5-pyridinedicarboxylate

Recrystallized from 2-propanol/cyclohexane. mp 107-9°.

Example 10

3-Methyl 5-(2-methylpropyl) 2-(fluoromethyl)-1,4-dihydro-6-methyl-4-(3-nitrophenyl)-3,5-pyridinedicarboxylate

Recrystallized from 2-propanol/cyclohexane. mp 101-2°.

Example 11

3-Ethyl 5-methyl 2-(fluoromethyl)-1,4-dihydro-6-methyl-4-(3-nitrophenyl)-3,5-pyridinedicarboxylate

Recrystallised from 2-propanol. mp 137-8°.

Example 12

Diethyl 2-(fluoromethyl)-1,4-dihydro-6-methyl-4-(2-(trifluoromethyl)phenyl)-3,5-pyridinedicarboxylate

Crystallised from hexane. mp 84-6°.

Example 13

Diethyl 4-(4-benzofurazanyl)-2-(fluormethyl)-1,4-dihydro-6-methyl-3,5-pyridinedicarboxylate

Recrystallised from methylene chloride/cyclohexane. mp 125-7°.

Example 14

Dimethyl 2-(fluoromethyl)-1,4-dihydro-6-methyl-4--(2,3,4,5,6-pentafluorophenyl)-3,5-pyridinedicarboxylate

Crystallised from cyclohexane. mp 148-50°.

Example 15

5-Methyl 3-(1-methylethyl) 2-(fluoromethyl)-1,4-dihydro-6-methyl-4-(3-nitrophenyl)-3,5-pyridinedicarboxylate

Recrystallised from methylene chloride/cyclohexane. mp 122-4°.

Example 16

5-Ethyl 3-methyl 4-(2,3-dichlorophenyl)-2-(fluoromethyl)-1,4-dihydro-6-methyl-3,5-pyridinedicarboxylate

Recrystallised from methylene chloride/cyclohexane. mp 124-5°.

Example 17

Diethyl 2-(fluoromethyl)-1,4-dihydro-6-methyl-4-(2-(methylthio)-3-pyridyl)-3,5-pyridinedicarboxylate

Recrystallised from cyclohexane/petroleum ether (60-80°). mp 92-4°.

Example 18

3-Methyl 5-(2-(methyl(phenylmethyl)amino)ethyl) 2-(fluoromethyl)-1,4-dihydro-6-methyl-4-(3-nitrophenyl)-3,5-pyridinedicarboxylate oxalate hemihydrate

The purified free base was converted into the oxalate which was obtained as a yellow solid after trituration with ether, mp 95° with decomposition, softens at about 70°.

Example 19

5-(2-Methoxyethyl) 3-(1-methylethyl) 4-(2,3-dichlorophenyl)-2-(fluoromethyl)-1,4-dihydro-6-methyl-3,5-pyridinedicarboxylate

Recrystallised from cyclohexane/petroleum ether (60-80°). mp 88-9°.

Example 20

5-(2-Cyanoethyl) 3-(1-methylethyl) 2-(fluoromethyl)-1,4-dihydro-6-methyl-4-(3-nitrophenyl)-3,5-pyridinedicarboxylate

Recrystallised from 2-propanol. mp 231-232.5°.

Example 21

3-(1-Methylethyl) 5-(2-(methylthio)ethyl) 2-(fluoromethyl)-1,4-dihydro-6-methyl-4-(3-nitrophenyl)-3,5-pyridinedicarboxylate

Recrystallised from 2-propanol/petroleum ether (60-80°). mp 109-111°.

Example 22

3-Methyl 5-(1-methylethyl) 4-(2-chloro-5-nitrophenyl)-2-(fluoromethyl)-1,4-dihydro-6-methyl-3,5-pyridinedicarboxylate

Recrystallised from 2-propanol/petroleum ether (60-80°). mp. 131-133°.

Example 23

3-Ethyl 5-methyl 4-(2,3-dichlorophenyl)-2-(fluoromethyl)-1,4-dihydro-6-methyl-3,5-pyridinedicarboxylate

Obtained as a solid by trituration with petroleum ether (60-80°). mp 99-101°.

Example 24

3-Methyl 5-(1-methylethyl) 2-(fluoromethyl)-1,4-dihydro-6-methyl-4-(2-methyl-5-nitrophenyl)-3,5-pyridinedicarboxylate

Recrystallised from 2-propanol/petroleum ether (60-80°), mp 100-1°.

Example 25

3-(2-Methoxyethyl) 5-(1-methylethyl) 2-(fluoromethyl)-1,4-dihydro-6-methyl-4-(3-nitrophenyl)-3,5-pyridinedicarboxylate

Recrystallised from cyclohexane as yellow crystals mp 112-4°.

Example 26

5-(2-Methoxyethyl) 3-(1-methylethyl) 2-(fluoromethyl)-1,4-dihydro-6-methyl-4-(3-nitrophenyl)-3,5-pyridinedicarboxylate

Recrystallised from cyclohexane-isopropanol as a

yellow solid mp 95-6°.

Example 27

3-Methyl 5-(1-methylethyl) 4-(2-chloro-3-(trifluoromethyl)phenyl)-2-(fluoromethyl)-1,4-dihydro-6-methyl-3,5-pyridinedicarboxylate

Recrystallised from petroleum ether (60-80°) mp 145-7°.

Example 28

5-(1-Methylethyl) 3-(tetrahydro-4H-pyran-4-yl) 2-(fluoromethyl)-1,4-dihydro-6-methyl-4-(3-nitrophenyl)-3,5-pyridinedicarboxylate

Recrystallised from petroleum ether (60-80°)/acetone, mp 128-30°.

Example 29

5-(1-Methylethyl) 3-(2-phenoxyethyl) 2-(fluoromethyl)-1,4-dihydro-6-methyl-4-(3-nitrophenyl)-3,5-pyridinedicarboxylate

$M^+$ 498; nmr ($CDCl_3$) $\delta$ 6.6(d,NH), 5.1(s,H).

Example 30

5-Methyl 3-(tetrahydro-4H-pyran-4-yl) 2-(fluoromethyl) 1,4-dihydro-6-methyl-4-(3-nitrophenyl)-3,5-pyridinedicarboxylate

Yellow prisms (acetone/petroleum ether 60-80°) mp 152-4°.

Example 31

5-Cyclohexyl 3-methyl 4-(2,3-dichlorophenyl)-2-(fluoromethyl)-1,4-dihydro-6-methyl-3,5-pyridinedicarboxylate

Orange prisms (petroleum ether 60-80°) mp 121-3°.

Example 32

5-Cyclopentyl 3-methyl 2-(fluoromethyl)-1,4-dihydro-6-methyl-4-(2-methyl-3-nitrophenyl)-3,5-pyridinedicarboxylate

mp 167-8°. (2-Propanol).

Example 33

3-Methyl 5-(1-methylethyl) 4-(2,3-dimethoxyphenyl)-2-(fluoromethyl)-1,4-dihydro-6-methyl-3,5-pyridinedicarboxylate

mp 89-91°. (Petroleum ether 60-80°/2-propanol).

Example 34

3-Methyl 5-(tetrahydro-4H-pyran-4-yl) 4-(2,3-dichlorophenyl)-2-(fluoromethyl)-1,4-dihydro-6-methyl-3,5-pyridinedicarboxylate

Orange prisms (acetone/petroleum ether 60-80°) mp 143-5°.

Example 35

5-Cyclopentyl 3-methyl 2-(fluoromethyl)-1,4-dihydro-6-methyl-4-(2-(trifluoromethyl)phenyl)-3,5-pyridinedicarboxylate

Yellow crystals (petroleum ether 40-60°) mp 122-3°.

Example 36

5-Cyclopentyl 3-methyl 4-(3-chloro-6-fluoro-2-(trifluoromethyl)phenyl)-2-(fluoromethyl)-1,4-dihydro-6-methyl-3,5-pyridinedicarboxylate

mp 184-6°. (2-Propanol/petroleum ether 60-80°).

Example 37

5-(1-Ethylpropyl) 3-methyl 4-(2,3-dichlorophenyl)-2-(fluoromethyl)-1,4-dihydro-6-methyl-3,5-pyridinedicarboxylate

Pale yellow prisms (petroleum ether 40-60°) mp 118-9°.

Example 38

3-Methyl 5-(1-methylethyl) 2-(fluoromethyl)-1,4-dihydro-4-(2-methoxy-3-nitrophenyl)-6-methyl-3,5-pyridinedicarboxylate

mp 105-6°. (2-Propanol/petroleum ether 60-80°).

Example 39

3-Methyl 5-(1-methylethyl) 2-(fluoromethyl)-1,4-dihydro-6-methyl-4-(2-(trifluoromethyl)phenyl)-3,5-pyridinedicarboxylate

Pale yellow crystals (hexane) mp 71-2°.

Example 40

3-Methyl 5-(1-methylethyl) 2-(fluoromethyl)-1,4-dihydro-6-methyl-4-(2-nitrophenyl)-3,5-pyridinedicarboxylate

Yellow crystals (petroleum ether 60-80°) mp 142-3°.

Example 41

3-Methyl 5-(1-methylethyl) 2-(fluoromethyl)-4-(2-fluorophenyl)-1,4-dihydro-6-methyl-3,5-pyridinedicarboxylate

Yellow crystals (petroleum ether 60-80°) mp 129-31°.

Example 42

3-Methyl 5-(1-methylethyl) 2-(fluoromethyl)-1,4-dihydro-6-methyl-4-(2-methylphenyl)-3,5-pyridinedicarboxylate

Pale yellow crystals (petroleum ether 60-80°) mp 94-5°.

Example 43

3-Methyl 5-(1-methylethyl) 4-(2-chlorophenyl)-2-(fluoromethyl)-1,4-dihydro-6-methyl-3,5-pyridinecarboxylate

Yellow crystals (petroleum ether 60-80°) mp 137-9°.

Example 44

3-Methyl 5-(1-methylethyl) 2-(fluoromethyl)-1,4-dihydro-6-methyl-4-(2-(methylthio)phenyl)-3,5-pyridinedicarboxylate

Example 45

3-Methyl 5-(1-methylethyl) 2-(fluoromethyl)-1,4-dihydro-6-methyl-4-(2-(methylsulphonyl)phenyl)-3,5-pyridinedicarboxylate

Example 46

3-Methyl 5-(1-methylethyl) 4-(3-chloro-2-methylphenyl)-2-(fluoromethyl)-1,4-dihydro-6-methyl-3,5-pyridinedicarboxylate

mp 73-5$^{o}$ (cyclohexane).

Example 47

3-Methyl 5-(1-methylethyl) 4-(2,3-dimethylphenyl)-2-(fluoromethyl)-1,4-dihydro-6-methyl-3,5-pyridinedicarboxylate

Example 48

3-Methyl 5-(1-methylethyl) 4-(3-cyanophenyl)-2-(fluoromethyl)-1,4-dihydro-6-methyl-3,5-pyridinedicarboxylate

Pale yellow crystals (2-propanol/petroleum ether 60-80$^{o}$) mp 117-8$^{o}$.

Example 49

3-Methyl 5-(1-methylethyl) 4-(3-chlorophenyl)-2-(fluoromethyl)-1,4-dihydro-6-methyl-3,5-pyridinedicarboxylate

Pale yellow crystals (petroleum ether 60-80$^{o}$) mp 107-9$^{o}$.

Example 50

Diethyl 1,2,3,4-tetrahydro-2-hydroxy-6-methyl-4-(3-nitrophenyl)-2-(trifluoromethyl)-3,5-pyridinedicarboxylate

3-Nitrobenzaldehyde (3.0g, 20 mmoles), ethyl 3-amino-2-butenoate (2.6g, 20 mmoles) and ethyl 4,4,4-trifluoro-3-oxobutanoate (2.92ml, 20 mmoles) were heated at reflux in ethanol (25ml) for 6 hours. The solvent was removed *in vacuo* and the residue crystallised by the addition of ether/petroleum ether (60-80°). The resulting solid was recrystallised from ether/petroleum ether (60-80°) to give the title compound as colourless crystals (1.9g) mp 120-1°.

Example 51

3-Ethyl 5-methyl 4-(2,3-dichlorophenyl)-1,2,3,4-tetrahydro-2-hydroxy-6-methyl-2-(trifluoromethyl)-3,5-pyridinedicarboxylate

Prepared by the method of Example 50. Two isomeric compounds were obtained. Isomer 1 recrystallised from cyclohexane mp 140-1°. Isomer 2 recrystallised from cyclohexane mp 118-9.5°.

Example 52

Diethyl 1,2,3,4-tetrahydro-2-hydroxy-6-methyl-4-(3-nitrophenyl)-2-(pentafluoroethyl)-3,5-pyridinedicarboxylate

Prepared by the method of Example 50. Two isomeric

compounds were obtained. Isomer 1 recrystallised from 2-propanol mp 103-4°. Isomer 2 recrystallised from 2-propanol mp 121-2°.

Example 53

5-Cyclopentyl 3-methyl 4-(2,3-dichlorophenyl)-2-(fluoromethyl)-1,4-dihydro-6-methyl-3,5-pyridinedicarboxylate

a) Cyclopentyl 2-(2,3-dichlorophenylmethylene)-3-oxobutanoate

A solution of 2,3-dichlorobenzaldehyde (2.5g, 14.3mmoles), cyclopentyl 3-oxobutanoate (2.42g, 14.3mmoles), piperidine (8 drops) and hexanoic acid (11 drops) in dry benzene (80ml) was heated at reflux for 4 hours using a Dean and Stark apparatus. The solution was allowed to cool to room temperature and the solvent removed *in vacuo* to leave the sub-title compound as an oil 5.1g.

b) A solution of the product of step a) (5.1g, 14.3mmoles) and methyl 3-amino-4-fluoro-2-butenoate (1.9g, 14.3mmoles) in dry tert-butanol (25ml) was heated to 60° (oil bath temperature) for 108 hours. The solution was allowed to cool to room temperature and the solvent removed *in vacuo*. Chromatography on silica eluting with dichloromethane afforded the title compound as a yellow oil which crystallises on addition of petroleum ether

(60-80°) to afford the title compound 1.5g mp 148-9°. (Identical with the product of Example 3).

Example 54

5-Cyclobutyl 3-methyl 4-(2,3-dichlorophenyl)-2-(fluoromethyl)-1,4-dihydro-6-methyl-3,5-pyridinedicarboxylate

a) Cyclobutyl 2-(2,3-dichlorophenylmethylene)-3-oxobutanoate

A solution of 2,3-dichlorobenzaldehyde (1.57g, 8.9mmoles), cyclobutyl 3-oxobutanoate (1.4g, 8.9mmoles), piperidine (8 drops) and hexanoic acid (11 drops) in dry benzene (100ml) was heated at reflux for 12 hours using a Dean and Stark apparatus. The solution was allowed to cool to room temperature and the solvent removed *in vacuo* to leave the crude sub-title compound as an oil 3.5g. Chromatography on silica eluting with petroleum ether (60-80°)/ethyl acetate mixtures afforded the sub-title compound as an oil, 1.7g.

b) The product of step a) (1.7g, 5.4mmoles) and methyl 3-amino-4-fluoro-2-butenoate (0.72g, 5.4mmoles) were mixed and heated to 95° (oil bath temperature) under an atmosphere of nitrogen for 6 hours. The oil was allowed to cool to room temperature. Chromatography on silica eluting with petroleum ether (60-80°)/ethyl acetate mixtures, afforded the title compound as a yellow solid,

which was recrystallised from petroleum ether (60-80°) to afford the title compound 0.37g, mp 148-9°.

Example 55

3-Methyl 5-(3-oxetanyl) 4-(2,3-dichlorophenyl)-2-(fluoromethyl)-1,4-dihydro-6-methyl-3,5-pyridinedicarboxylate

a) (3-Oxetanyl) 2-(2,3-dichlorophenylmethylene)-3-oxobutanoate

A solution of 2,3-dichlorobenzaldehyde (1.33g, 7.6mmoles), 3-oxetanyl 3-oxobutanoate (1.2g, 7.6mmoles), piperidine (6 drops) and hexanoic acid (8 drops) in dry benzene (80ml) was heated at reflux using a Dean and Stark apparatus. The solution was allowed to cool to room temperature and the solvent removed *in vacuo* to leave the sub-title compound as an oil 2.9g.

b) A solution of the product of step a) (2.9g, 7.6mmoles) and methyl 3-amino-4-fluoro-2-butenoate (1g, 7.6mmoles) in dry tert-butanol (20ml) was heated to 60° (oil bath temperature) for 16 hours. The solution was allowed to cool to room temperature and the solvent removed *in vacuo*. Chromatography on silica, eluting with dichloromethane/ethyl acetate mixtures afforded the title compound as an oil which crystallised on addition of petroleum ether (60-80°). The solid was recrystallised from petroleum ether (60-80°)/acetone to afford the

title compound 1.03g, mp 155-6°.

Example 56

Diethyl 1,2,3,4-tetrahydro-2-hydroxy-6-methyl-4-(3-nitrophenyl)-2-(trichloromethyl)-3,5-pyridinedicarboxylate

a) Ethyl 4,4,4-trichloro-2-(3-nitrophenylmethylene)--3-oxobutanoate

3-Nitrobenzaldehyde (7.55g, 50mmoles), ethyl 4,4,4-trichloro-3-oxobutanoate (18.33g, 59.5mmoles), piperidine (0.66ml) and hexanoic acid (0.33ml) were heated at reflux in toluene (130ml) for 48 hours using a Dean and Stark apparatus. The mixture was cooled, evaporated to dryness *in vacuo* and crystallised by trituration with ethyl acetate/petroleum ether (60-80°). Recrystallisation from 2-propanol gave the sub-title compound (5.3g) mp 105.5-7°.

b) Diethyl 1,2,3,4-tetrahydro-2-hydroxy-6-methyl-4-(3-nitrophenyl)-2-(trichloromethyl)-3,5-pyridinedicarboxylate

Ethyl 4,4,4-trichloro-2-(3-nitrophenylmethylene)-3-oxobutanoate (7.19g, 0.02mmoles) and ethyl 3-amino-2-butenoate (2.53g) were heated at 60° for 24 hours in tert-butanol (60ml). The solvent was evaporated and the residue chromatographed on silica eluting with ether/petroleum ether (60-80°) mixtures to give the

title compound (4.04g). Recrystallised from 2-propanol. mp 125-6.5°.

Example 57

3-Methyl 5-((S)-2,2,2-trichloro-1-phenylethyl) 4-(2,3-dichlorophenyl)-2-(fluoromethyl)-1,4-dihydro-6-methyl-3,5-pyridinedicarboxylate

a) (S)-2,2,2-Trichloro-1-phenylethyl 2-(2,3-dichlorophenylmethylene)-3-oxobutanoate

(S)-2,2,2-Trichloro-1-phenylethyl 3-oxobutanoate (8.6g, 27.5mmoles) and 2,3-dichlorobenzaldehyde (4.82g, 27.5mmoles) in dry benzene (100ml) were heated at reflux for 5 hours with hexanoic acid (25 drops) and piperidine (8 drops) in a Dean and Stark apparatus. The solvent was evaporated and the residue dissolved in ethyl acetate (200ml), washed with saturated sodium bicarbonate, 2% aqueous sodium bisulphite solution and brine, dried ($MgSO_4$) and the solvent removed in vacuo. The sub-title compound was obtained as a yellow oil. HPLC and nmr indicate 2:1 mixture of geometric isomers.

b) 3-Methyl 5-((S)-2,2,2-trichloro-1-phenylethyl) 4-(2,3-dichlorophenyl)-2-(fluoromethyl)-1,4-dihydro-6-methyl-3,5-pyridinedicarboxylate Single diastereomers A and B

(S)-2,2,2-Trichloro-1-phenylethyl 2-(2,3-dichlorophenylmethylene)-3-oxobutanoate (10.8g, 23mmoles) and methyl 3-amino-4-fluoro-2-butenoate (3.7g, 28mmoles)

were heated at 55° in dry tert- butanol (50ml) for 68 hours. The solvent was removed and the residue purified and separated into single diastereomers by HPLC eluting with methylene chloride/petroleum ether (60-80°) mixtures.

First eluted: diastereomer A, recrystallised from cyclohexane/petroleum ether (60-80°) mp 167.5-8° $[\alpha]^{25}_{D}$ +17.5° (c, 0.1 in ethanol).

Second eluted: diastereomer B, recrystallised from petroleum ether (60-80°) mp 141-3° $[\alpha]^{25}_{D}$ -150.1° (c, 0.1 in ethanol).

<u>Example 58</u>

<u>3-Methyl 5-(2,2,2-trichloro-1-phenylethyl) 4-(2,3-dichlorophenyl)-2-(fluoromethyl)-1,4-dihydro-6-methyl-3,5-pyridinedicarboxylate Diastereomeric pairs A and B</u>

Prepared by the method of Example 57 and separated by HPLC using methylene chloride/petroleum ether (60-80°) mixtures.

First eluted: diastereomeric pair A. Recrystallised from cyclohexane/petroleum ether (60-80°) mp 203-4°.

Second eluted: diastereomeric pair B. Recrystallised from cyclohexane/petroleum ether (60-80°) mp 176-176.5°.

The compounds of Examples 59 to 73 were prepared using appropriate starting materials and the method

described in Examples 53-58.

Example 59

Diethyl 2-(difluoromethyl)-1,4-dihydro-6-methyl-4-(3-nitrophenyl)-3,5-pyridinedicarboxylate

mp 148°-9° (2-propanol).

Example 60

Diethyl 1,4-dihydro-2-methyl-6-methylthio-4-(3-nitrophenyl)-3,5-pyridinedicarboxylate

mp 123-5° (2-propanol).

Example 61

Diethyl 1,4-dihydro-2-(4-methoxyphenyl)-6-methyl-4-(3-nitrophenyl)-3,5-pyridinedicarboxylate

mp 166-7° (2-Propanol).

Example 62

Diethyl 2-(dichloromethyl)-1,4-dihydro-6-methyl-4-(3-nitrophenyl)-3,5-pyridinedicarboxylate

Recrystallised from isopropanol as yellow crystals mp 139-141°.

Example 63

Diethyl 1,4-dihydro-2-methyl-6-phenyl-4-(2-(trifluoromethyl)phenyl)-3,5-pyridinedicarboxylate

Recrystallisation from 2-propanol gave the title compound (3.7g) mp 149-150°.

Example 64

5-Methyl 3-(1-methylethyl) 4-(4-benzofurazanyl)-1,4-dihydro-2-methyl-6-phenyl-3,5-pyridinedicarboxylate

Recrystallised from 2-propanol to give the title compound mp 198-200°.

Example 65

5-Methyl 3-(1-methylethyl) 1,4-dihydro-2-methyl-6-phenyl-4-(2-(trifluoromethyl)phenyl)-3,5-pyridinedicarboxylate

Recrystallised from petroleum ether (60-80°) mp 111-2°.

Example 66

5-Ethyl 3-methyl 4-(2,3-dichlorophenyl)-1,2,3,4-tetrahydro-2-hydroxy-6-methyl-2-phenyl-3,5-pyridinedicarboxylate

The title compound was obtained as a white solid. Nmr ($D_6$-DMSO) $\delta$ 6.0(s,1H), 4.9(d,1H,J=11Hz), 0.7(t,3H, J=7Hz).

Example 67

Diethyl 4-(4-benzofurazanyl)-2-diethoxymethyl-1,4,5,6-tetrahydro-6-hydroxy-6-(triflurومethyl)-3,5-pyridinedicarboxylate

The title product was obtained as an oil. $M^+$ 531.

Example 68

5-(1-(Diphenylmethyl)-3-azetidinyl) 3-methyl 4-(2,3-dichlorophenyl)-2-(fluoromethyl)-1,4-dihydro-6-methyl-3,5-pyridinedicarboxylate

Pale yellow solid (petroleum ether 60-80°) mp 163-5°.

Example 69

3-Methyl 5-(1-(phenylmethyl)-4-piperidinyl) 4-(2,3-dichlorophenyl)-2-(fluoromethyl)-1,4-dihydro-6-methyl-3,5-pyridinedicarboxylate

Pale yellow solid. mp 118-20°.

Example 70

5-(1,1-Dimethylethyl) 3-methyl 4-(2,3-dichlorophenyl) -2-(fluoromethyl)-1,4-dihydro-6-methyl-3,5-pyridinedicarboxylate

Pale yellow solid (petroleum ether 60-80°) mp 141°.

Example 71

3-Methyl 5-(1-methyl-1-phenylethyl) 4-(2,3-dichlorophenyl)-2-(fluoromethyl)-1,4-dihydro-6-methyl-3,5-pyridinedicarboxylate

Colourless solid (acetone-petroleum ether 60-80°). mp 173-5°.

Example 72

3-Methyl 5-(1-methylcyclopentyl) 4-(2,3-dichlorophenyl)-2-(fluoromethyl)-1,4-dihydro-6-methyl-3,5-pyridinedicarboxylate

Colourless solid (petroleum ether 60-80°). mp 111°.

Example 73

3-Methyl 5-(2,2,2-trichloro-1-phenylethyl) 2-(fluoromethyl)-1,4-dihydro-6-methyl-4-(3-nitrophenyl)-3,5-pyridinedicarboxylate

Diastereomers obtained as yellow foam. $M^+$ 562/560/558/556. Nmr ($CDCl_3$) $\delta$ 5.24 and 5.26 (2xs,1H), 6.32 and 6.34 (2xs,1H).

Example 74

Diethyl 2-(fluoromethyl)-6-formyl-1,4-dihydro-4-(3-nitrophenyl)-3,5-pyridinedicarboxylate

Ethyl 4,4-diethoxy-2-(3-nitrophenylmethylene)-3-oxobutanoate (21.75g, 62mmoles) and ethyl 3-amino-4-fluoro-2-butenoate (9.17g, 68mmoles) were heated at 125° for 1.5 hours. The reaction mixture was dissolved in ethyl acetate (150ml), washed with water and saturated brine, dried ($MgSO_4$) and the solvent was removed *in vacuo*. The residue was dissolved in tetrahydrofuran (195ml) and 50% aqueous hydrochloric acid (292ml) was added slowly. After 30 minutes the reaction mixture was extracted with ethyl acetate and the organic extract washed with saturated aqueous sodium bicarbonate, water, dried ($MgSO_4$) and the solvent was removed *in vacuo*. The residue was chromatographed on silica eluting with ethyl acetate/petroleum ether (60-80°) mixtures. Crystallisation from 2-propanol gave the title compound

(4.6g), mp 88-90$^{o}$.

Example 75

3-Methyl 5-(1-methylethyl) 4-(4-benzofurazanyl)-1,2,3,4-tetrahydro-2-hydroxy-6-methyl-2-phenyl-3,5-pyridinedicarboxylate

4-Benzofurazancarboxaldehyde (2.96g, 20mmoles), methyl beta-oxobenzenepropanoate (3.56g, 20mmoles), piperidine (0.05ml) and hexanoic acid (0.13ml) were heated at reflux for 3 hours in benzene (50ml) using a Dean and Stark apparatus. The reaction was cooled, diluted with ethyl acetate and washed in turn with water, brine and saturated sodium bicarbonate and dried ($Na_2SO_4$). The solvent was removed in vacuo and the residue dissolved in ethanol (6ml). 1-Methylethyl 3-amino-2-butenoate (3.0g) and diethylamine (0.6ml) were added and the mixture heated at 60$^{o}$ for 34 hours. The reaction mixture was cooled, evaporated to dryness in vacuo and the residue was dissolved in 2-propanol and treated with charcoal. The charcoal was filtered off and the title compound (1.1g) was obtained on addition of cyclohexane mp 132-4$^{o}$.

Example 76

Diethyl 2-amino-6-(fluoromethyl)-1,4-dihydro-4-(3-nitrophenyl)-3,5-pyridinedicarboxylate

Ethyl 4-fluoro-2-(3-nitrophenylmethylene)-3-oxobutanoate (0.6g, 2.1mmoles) and ethyl 3,3-diamino-2-propenoate hydrochloride (0.34g, 12.0mmoles) were heated at reflux in ethanol (10ml) and a solution of sodium (0.05g) in ethanol (5ml) was added over one hour. The resulting solution was heated at reflux for a further 10 minutes and then filtered hot. The ethanolic solution was evaporated to dryness in vacuo and the resulting solid triturated with 2-propanol. The resulting solid was chromatographed on silica eluting with ether/petroleum ether (60-80$^{\circ}$) mixtures to give pure title compound, mp 177-8$^{\circ}$.

Example 77

Diethyl 2-(fluoromethyl)-1,4,5,6-tetrahydro-6-hydroxy-4-(3-nitrophenyl)-6-phenyl-3,5-pyridinedicarboxylate and Diethyl 2-(fluoromethyl)-1,4-dihydro-4-(3-nitrophenyl)-6-phenyl-3,5-pyridinedicarboxylate

Ethyl alpha-(3-nitrophenylmethylene)-beta-oxobenzenepropanoate (1.66g, 16.3mmoles), ethyl 3-amino-4-fluoro-2-butenoate (0.75g, 15.6mmoles) and piperidine (0.06ml) were heated at 60$^{\circ}$ in ethanol (1ml) for 72 hours. The reaction mixture was cooled and diluted with ethanol. The solid was filtered off, dried and then chromatographed on silica eluting with ethyl acetate/petroleum ether (60-80$^{\circ}$) mixtures to give the hydroxy compound (0.43g) mp 188-90$^{\circ}$(dec).

The ethanolic mother liquors were evaporated to

dryness in vacuo, dissolved in methylene chloride and pyridine (0.58ml), and trifluoroacetic anhydride (0.48ml) was added with stirring. After 16 hours, the solution was washed with 5% aqueous acetic acid (3 x 10ml), and saturated sodium bicarbonate, dried ($Na_2SO_4$) and the solvent was removed in vacuo. The residue was chromatographed on silica eluting with ethyl acetate/petroleum ether (60-80°) mixtures. Recrystallisation from 2-propanol gave the dihydropyridine (0.31g), mp 151-2°.

Example 78

3-Ethyl 5-methyl 4-(2,3-dichlorophenyl)-1,4-dihydro-2-methyl-6-phenyl-3,5-pyridinedicarboxylate

Ammonia (0.5ml, d =0.88) was added to a solution of methyl alpha-(2,3-dichlorophenylmethylene)-beta-oxobenzenepropanoate (2g, 7.3mmoles) and ethyl 3-amino-2-butenoate (0.77g, 6.0mmoles) in tert. butanol (8ml) at 60°. The reaction was maintained at this temperature for 16 hours. The solvent was removed in vacuo and the residue was chromatographed on silica eluting with ethyl acetate/petroleum ether (60-80°) mixtures. The title compound (0.25g) was obtained after crystallisation from 2-propanol mp 185-6°.

Example 79

Diethyl 1,4-dihydro-2-methyl-4-(3-nitrophenyl)-6-(4-nitrophenyl)-3,5-pyridinedicarboxylate

Trifluoroacetic anhydride (0.65ml, 4.63mmoles) was added with stirring to pyridine (0.75ml, 9.26mmoles) and diethyl 1,2,3,4-tetrahydro-2-hydroxy-6-methyl-4-(3-nitrophenyl)-2-(4-nitrophenyl)-3,5-pyridinedicarboxylate (2.31g, 4.63mmoles) in methylene chloride (60ml). After stirring for 2.5 hours, the solution was washed with water, dilute hydrochloric acid (x3), water, saturated sodium bicarbonate solution, water and dried ($Na_2SO_4$). The solvent was removed *in vacuo* and the residue recrystallised from ethanol to give the title compound (1.77g) as a yellow solid, mp 176-7$^o$.

The compounds of Examples 80 to 86 were prepared using appropriate starting materials and the method of Example 79.

Example 80

Diethyl 2-(3,4-dichlorophenyl)-1,4-dihydro-6-methyl-4-(3-nitrophenyl)-3,5-pyridinedicarboxylate

Yellow solid mp 153-6$^o$ (ethanol).

Example 81

Diethyl 2-(4-chlorophenyl)-1,4-dihydro-6-methyl-4-(3-nitrophenyl)-3,5-pyridinedicarboxylate

Yellow solid mp 158-60$^o$ (ethanol).

Example 82

Diethyl 1,4-dihydro-2-methyl-4-(3-nitrophenyl)-6-(trifluoromethyl)-3,5-pyridinedicarboxylate

Yellow solid mp 93-5° (ether-petroleum ether 60-80°).

Example 83

Diethyl 2-(3-chlorophenyl)-1,4-dihydro-6-methyl-4-(3-nitrophenyl)-3,5-pyridinedicarboxylate

Recrystallised from 2-propanol. mp 160-1°.

Example 84

Diethyl 1,4-dihydro-2-methyl-4-(3-nitrophenyl)-6-(pentafluoroethyl)-3,5-pyridinedicarboxylate

Obtained pure after chromatography. mp 88-9°.

Example 85

5-Ethyl 3-methyl 4-(2,3-dichlorophenyl)-1,4-dihydro-2-methyl-6-(trifluoromethyl)-3,5-pyridinedicarboxylate

Recrystallised from cyclohexane, mp 101-2°.

Example 86

Diethyl 4-(4-benzofurazanyl)-2-(diethoxymethyl)-1,4-dihydro-6-(trifluoromethyl)-3,5-pyridinedicarboxylate

Obtained as an oil. Nmr ($CDCl_3$) δ 6.1(s,H), 5.6(s,H).

Example 87

Diethyl 1,4-dihydro-2-methyl-4-(3-nitrophenyl)-6-(trichloromethyl)-3,5-pyridinedicarboxylate

a) Diethyl 3,4-dihydro-2-methyl-4-(3-nitrophenyl)-6-(trichloromethyl)-3,5-pyridinedicarboxylate

Diethyl 1,2,3,4-tetrahydro-2-hydroxy-6-methyl-4-(3-nitrophenyl)-2-(trichloromethyl)-3,5-pyridinedicarboxylate (3.0g, 6.05mmoles) was dissolved in dry methylene chloride (150ml) and diethylaminosulphur trifluoride (1.5ml) was added. After 1 hour the solution was diluted with methylene chloride and washed in turn with dilute hydrochloric acid and saturated sodium bicarbonate solution. After drying ($MgSO_4$), the solvent was removed *in vacuo* to give the sub-title compound (2.82g) as an oil. Nmr ($D_6$-DMSO) $\delta$ 4.8 (s,H), 4.4 (s,H).

b) Diethyl 1,4-dihydro-2-methyl-4-(3-nitrophenyl)-6-(trichloromethyl)-3,5-pyridinedicarboxylate

The product of step a) (2.67g) was dissolved in methylene chloride (20ml) and triethylamine (0.5ml) was added. After 18 days at room temperature, the solvent was evaporated and the residue chromatographed on silica eluting with methylene chloride. The title compound (0.65g) was obtained after crystallisation from 2-propanol mp 113-5$^{\circ}$. Nmr ($D_6$-DMSO) $\delta$ 9.0 (s,H), 4.9 (s,H).

Example 88

Diethyl 1,4-dihydro-2-methyl-6-(methylsulphinyl)-4-(3-nitrophenyl)-3,5-pyridinedicarboxylate Isomers I and II

Peracetic acid (6.8ml of 1M solution in methanol) was added to a solution of diethyl 1,4-dihydro-2-methyl-6-

(methylthio)-4-(3-nitrophenyl)-3,5-pyridinedicarboxylate (2.77g 6.8mmoles) in methylene chloride (150ml) at -78°. The reaction mixture was allowed to reach room temperature and was then stirred for 30 minutes. Saturated aqueous sodium bicarbonate (150ml) was added and the organic layer separated, dried ($Na_2SO_4$) and the solvent removed _in vacuo_. The residue was chromatographed on silica, eluting with ether/petroleum ether (60-80°) mixtures. The two isomers were separated and recrystallised from 2-propanol.

Diastereomer I yellow crystals mp 143-4° (0.84g).

Diastereomer II yellow crystals mp 133-5° (1.25g).

Example 89

Diethyl 2-aminocarbonyl-1,4-dihydro-6-methyl-4-(3-nitrophenyl)-3,5-pyridinedicarboxylate

A solution of 3,5-diethyl 1,4-dihydro-6-methyl-4-(3-nitrophenyl)-2,3,5-pyridinetricarboxylate (4.0g; 10.3mmoles), 1,1'-carbonyldiimidazole (1.75g; 10.8mmoles) in dry dichloromethane (180ml) was stirred at room temperature under an atmosphere of dry nitrogen. After 2 hours a yellow suspension had formed, ammonia solution (20ml, d=0.88) was added and the 2-phase mixture left stirring for 16 hours.

Saturated brine (100ml) was added, the organic solution was separated, washed with 15% aq. sodium

hydroxide solution, saturated brine, water and dried ($MgSO_4$).

Evaporation of the solvent was followed by chromatography on silica (150g) using ethyl acetate/petroleum ether (60-80$^o$) as eluent.

The title compound was obtained as a white solid which was recrystallised from 2-propanol to give a white powder (0.8g) mp 166-8$^o$.

Example 90

Diethyl 2-(dimethylaminocarbonyl)-1,4-dihydro-6-methyl-4-(3-nitrophenyl)-3,5-pyridinedicarboxylate

Thionyl chloride (0.05ml) was added to a solution of 3,5-diethyl 1,4-dihydro-6-methyl-4-(3-nitrophenyl)-2,3,5-pyridinetricarboxylate (0.25g, 0.62mmoles) in methylene chloride (10ml) containing dimethylformamide (1 drop). After 2 hours at room temperature further thionyl chloride (0.05ml) was added and the solution was refluxed for 30 mins. After cooling to room temperature 10% dimethylamine in benzene (1 ml) was added and the mixture stirred for 30 mins. The solvent was evaporated and the residue dissolved in dilute hydrochloric acid and ether. The organic extract was washed with brine, dried ($Na_2SO_4$) and the solvent removed *in vacuo* to leave the title compound (0.2g). $M^+$ 431; nmr ($CDCl_3$) $\delta$ 5.12 (s,H), 3.05 (s,3H), 2.95 (s,3H).

Example 91

Diethyl 1,4-dihydro-2-(1H-imidazol-1-ylcarbonyl)-6-methyl-4-(3-nitrophenyl)-3,5-pyridinedicarboxylate and Diethyl 1,4-dihydro-2-methyl-6-(4-morpholinylcarbonyl)-4-(3-nitrophenyl)-3,5-pyridinedicarboxylate

A solution of 3,5-diethyl 1,4-dihydro-6-methyl-4-(3-nitrophenyl)-2,3,5-pyridinetricarboxylate (2.5g, 6.2mmoles) and 1,1'-carbonyldiimidazole (1.2g, 7.4mmoles) in methylene chloride (100ml) was stirred at room temperature for 4 hours. Morpholine (1.08ml, 12.4mmoles) was added, the mixture stirred overnight and then poured onto 10% hydrochloric acid. The organic layer was separated, washed with 10% hydrochloric acid, brine, saturated sodium bicarbonate, brine and dried ($Na_2SO_4$). The solvent was evaporated and the residue chromatographed on silica eluting with ethyl acetate/petroleum ether (60-80$^{o}$) mixtures. The imidazolyl-carbonyl (0.24g) compound was eluted first ($M^+$ 454).

Further elution afforded the morpholinylcarbonyl compound (0.4g) ($M^+$ 473).

Example 92

Diethyl 2-(aminothioxomethyl)-1,4-dihydro-6-methyl-4-(3-nitrophenyl)-3,5-pyridinedicarboxylate

Hydrogen sulphide was bubbled through a solution of

diethyl 2-cyano-1,4-dihydro-6-methyl-4-(3-nitrophenyl)-3,5-pyridinedicarboxylate (1g, 2.6mmoles) in triethylamine (0.36ml, 2.6mmoles) and pyridine (20ml) at room temperature for 2 hours. The solution was degassed with nitrogen and poured into water (300ml). After stirring for 2 hours, the precipitate was filtered off, dissolved in methylene chloride and dried ($Na_2SO_4$). The solvent was removed *in vacuo* and the residue triturated with $CCl_4$ and filtered to give the title compound (0.5g). $M^+$ 419; ($CDCl_3$) $\delta$ 9.3 (s,$NH_2$), 5.2 (s,H).

Example 93

Diethyl 1,4-dihydro-2-(imino(methylthio)methyl)-6-methyl-4-(3-nitrophenyl)-3,5-pyridinedicarboxylate hydroiodide

Methyl iodide (0.06ml, 0.96mmoles) was added to a solution of diethyl 2-(aminothioxomethyl)-1,4-dihydro-6-methyl-4-(3-nitrophenyl)-3,5-pyridinedicarboxylate (0.2g, 0.48mmoles) in methanol (10ml). After stirring for 16 hours at room temperature methyl iodide (0.1ml) was added and the stirring continued for 1 day. The solvent was evaporated and the residue crystallised on addition of ether. The hydroscopic solid was filtered and dried *in vacuo* to give the title compound (0.17g).

Nmr ($CDCl_3$) $\delta$ 5.9 (s,H), 2.9 (s,SMe).

Example 94

Diethyl 2-(fluoromethyl)-1,4-dihydro-6-methyl-4-(3-nitrophenyl)-3,5-pyridinedicarboxylate

Ethyl 4-fluoro-3-oxobutanoate (1.45g, 10mmoles), ethyl 2-(3-nitrophenylmethylene)-3-oxobutanoate (2.63g, 10mmoles) and aqueous ammonia (1.1ml, d 0.88) were heated at reflux in ethanol (15ml) for 6 hours. The solvent was removed *in vacuo* and the residue purified by chromatography on silica eluting with petroleum ether (60-80$^{o}$)/ether mixtures. Recrystallisation from ether/petroleum ether (60-80$^{o}$) gave the title compound (1.1g) as yellow crystals mp 139-41$^{o}$.

The compounds of Examples 95 to 104 were prepared using appropriate starting materials and the method of Example 94.

Example 95

Di-(2-propoxyethyl) 2-(fluoromethyl)-1,4-dihydro-6-methyl-4-(3-nitrophenyl)-3,5-pyridinedicarboxylate

Recrystallised from ether-hexane as a yellow solid mp 52-3$^{o}$.

Example 96

Diethyl 1,2,3,4-tetrahydro-2-hydroxy-6-methyl-4-(3-nitrophenyl)-2-(4-nitrophenyl)-3,5-pyridinedicarboxylate

Recrystallised from ethanol as yellow needles mp 196-7$^{o}$.

Example 97

5-Methyl 3-(1-methylethyl) 1,4-dihydro-2-methyl-4-(3-nitrophenyl)-6-phenyl-3,5-pyridinedicarboxylate

mp 184.5-185.5° (2-propanol).

Example 98

Diethyl 1,2,3,4-tetrahydro-2-hydroxy-6-methyl-4-(3-nitrophenyl)-2-phenyl-3,5-pyridinedicarboxylate

m.p. 213-5°. (Ethanol).

Example 99

Diethyl 2-(3,4-dichlorophenyl)-1,2,3,4-tetrahydro-2-hydroxy-6-methyl-4-(3-nitrophenyl)-3,5-pyridinedicarboxylate

White solid mp 188-9°. (Ethanol).

Example 100

Diethyl 2-(4-chlorophenyl)-1,2,3,4-tetrahydro-2-hydroxy-6-methyl-4-(3-nitrophenyl)-3,5-pyridinedicarboxylate

Yellow solid mp 175-8°. (Ethanol).

Example 101

Diethyl 1,4-dihydro-2-methyl-6-(4-methylphenyl)-4-(3-nitrophenyl)-3,5-pyridinedicarboxylate

mp 149-50°. (Ethanol).

Example 102

3-Methyl 5-(1-methylethyl) 1,2,3,4-tetrahydro-2-hydroxy-6-methyl-4-(3-nitrophenyl)-2-phenyl-3,5-pyridinedicarboxylate

mp 134-5°. (2-Propanol).

Example 103

Diethyl 2-(3-chlorophenyl)-1,2,3,4-tetrahydro-2-hydroxy-6-methyl-4-(3-nitrophenyl)-3,5-pyridinedicarboxylate

mp 212-4°. (Ethanol).

Example 104

Diethyl 2-(2-furanyl)-1,4-dihydro-6-methyl-4-(3-nitrophenyl)-3,5-pyridinedicarboxylate

mp 130-1° (2-propanol).

Example 105

3-Methyl 5-(1-methylethyl) 2-(difluoromethyl)-1,4-dihydro-6-methyl-4-(3-nitrophenyl)-3,5-pyridinedicarboxylate

Diethylaminosulphur trifluoride (0.64ml, 5.1mmoles) was added to a stirred solution at -10° of 3-methyl 5-(1-methylethyl) 2-formyl-1,4-dihydro-6-methyl-4-(3-nitrophenyl)-3,5-pyridinedicarboxylate (2g, 5.2mmoles) in dry methylene chloride (20ml). After stirring for 2 hours at -10° and 1 hr at room temperature, diethylaminosulphur trifluoride (0.2ml) was added and the stirring continued for a further hour. The reaction mixture was poured into aqueous sodium bicarbonate (100ml) and extracted with methylene chloride (2 x 100ml). The organic extracts were washed with water (2x) and brine, dried ($MgSO_4$) and the solvent was evaporated.

Chromatography on silica eluting with ethyl acetate/petroleum ether (60-80°) mixtures, followed by crystallisation from 2-propanol gave the title compound (0.57g). mp 140-1°.

Example 106

5-Cyclopentyl 3-methyl 2-(difluoromethyl)-1,4-dihydro-6-methyl-4-(2-methyl-3-nitrophenyl)-3,5-pyridinedicarboxylate

5-Cyclopentyl 3-methyl 2-formyl-1,4-dihydro-6-methyl-4-(2-methyl-3-nitrophenyl)-3,5-pyridinedicarboxylate (0.62g, 1.45mmoles) was dissolved in dry methylene chloride (6ml) and then cooled to 0°. Diethylaminosulphur trifluoride (180μl, 1.45mmoles) was added and the reaction mixture stirred at room temperature for 4 hours. The solvent was removed *in vacuo* and the residue chromatographed on silica eluting with ether/petroleum ether (60-80°) mixtures. The title compound (0.22g) was obtained on evaporation of pure fractions mp 154-6°.

Example 107

3-Methyl 5-(1-methylethyl) 4-(2,3-dichlorophenyl)-2-(difluoromethyl)-1,4-dihydro-6-methyl-3,5-pyridinedicarboxylate

3-Methyl 5-(1-methylethyl) 4-(2,3-dichlorophenyl)-2-formyl-1,4-dihydro-6-methyl-3,5-pyridinedicarboxylate

(1.5g, 3.6mmoles) was dissolved in dry methylene chloride (20ml) and cooled to -60°. Diethylaminosulphur trifluoride (0.59g, 3.6mmoles) was added and the stirred mixture was allowed to reach room temperature. After 2 hours, the solvent was removed in vacuo and the residue chromatographed on silica eluting with methylene chloride/ethyl acetate mixtures. The title compound (0.6g) was obtained, after crystallisation from 2-propanol. mp 156-7°.

Example 108

Diethyl 2-(fluoromethyl)-1,4-dihydro-6-methyl-4-(3-nitrophenyl)-3,5-pyridinedicarboxylate

Diethyl 1,4-dihydro-2-(hydroxymethyl)-6-methyl-4-(3-nitrophenyl)-3,5-pyridinedicarboxylate (0.1g, 0.25mmoles) in dry methylene chloride (5ml) was added to a stirred solution at -60° of diethylaminosulphur trifluoride (0.068ml, 0.55mmoles) in dry methylene chloride (10ml) over 10 minutes. The reaction mixture was allowed to reach room temperature over 2.5 hours, poured into aqeuous sodium bicarbonate (15ml) and the aqueous layer extracted with methylene chloride (2x). The organic extracts were washed with water, dried ($MgSO_4$) and the solvent was evaporated. The residue was chromatographed on silica eluting with ethyl acetate/methylene chloride mixtures to give the title

compound (0.015g); identical with that prepared in Example 94.

The compounds of Examples 109 and 110 were prepared using appropriate starting materials and the method described in Examples 105-107.

Example 109

3-(2-Methoxyethyl) 5-(1-methylethyl) 4-(2,3-dichlorophenyl)-2-(difluoromethyl)-1,4-dihydro-6-methyl-3,5-pyridinedicarboxylate

mp. 127-128°. (2-Propanol).

Example 110

3-(2-Methoxyethyl) 5-(1-methylethyl) 2-(difluoromethyl)-1,4-dihydro-6-methyl-4-(3-nitrophenyl)-3,5-pyridinedicarboxylate

mp 146-7°. (2-Propanol).

Example 111

Diethyl 4-(4-benzofurazanyl)-2-formyl-1,4-dihydro-6-(trifluoromethyl)-3,5-pyridinedicarboxylate

To a solution of diethyl 4-(4-benzofurazanyl)-2-(diethoxymethyl)-1,4-dihydro-6-(trifluoromethyl)-3,5-pyridinedicarboxylate (7.6g, 14.5mmoles) in tetrahydrofuran (100ml) was added 25% aqueous hydrochloric acid solution (100ml) and the resulting solution heated at reflux. After 1.5 hours the cooled solution was poured into ethyl acetate (200ml). The organic phase was

separated and washed with water, saturated aqueous sodium bicarbonate solution, brine and dried ($MgSO_4$). Evaporation of the solvent left an oil (7.5g) which was purified by chromatography on silica (300g) using ether-petroleum ether (60-80°) as eluent. The major component was obtained as an oil which gave a solid on trituration with 2-propanol. Recrystallisation from 2-propanol gave the title compound (0.45g) as yellow crystals mp 94-5°.

Example 112

Diethyl 4-(4-benzofurazanyl)-1,4-dihydro-2-(hydroxymethyl)-6-trifluoromethyl-3,5-pyridinedicarboxylate

A solution of diethyl 4-(4-benzofurazanyl)-2-formyl-1,4-dihydro-6-(trifluoromethyl)-3,5-pyridinedicarboxylate (1.1g; 2.5mmoles) in dry ethanol (90ml) was cooled to 0° and sodium borohydride (0.14g; 3.7mmoles) was added portionwise over 3 minutes. After 10 minutes, 10% aqueous hydrochloric acid was added dropwise to pH3, and the mixture concentrated in vacuo at room temperature. The resulting yellow oil was dissolved in ether (50ml) and saturated aqueous sodium bicarbonate was added to pH9. The organic solution was separated, washed with saturated aqueous sodium bicarbonate solution, water, brine and dried ($MgSO_4$). Evaporation of the solvent left a yellow oil which was crystallised from 2-propanol to give the

title compound (0.5g) mp 110-11°.

The compound of Example 113 was prepared using appropriate starting materials and the method of Example 112.

Example 113

Diethyl 2-(fluoromethyl)-1,4-dihydro-6-hydroxymethyl-4-(3-nitrophenyl)-3,5-pyridinedicarboxylate

mp 149-50°. (2-Propanol).

Example 114

Diethyl 2-cyano-6-(fluoromethyl)-1,4-dihydro-4-(3-nitrophenyl)-3,5-pyridinedicarboxylate

(a) Diethyl 2-(2,4-dinitrophenoxyiminomethyl)-6-(fluoromethyl)-1,4-dihydro-4-(3-nitrophenyl)-3,5-pyridinedicarboxylate

The compound of Example 74 (0.2g, 10.5mmoles) and O-(2,4-dinitrophenyl)hydroxylamine (0.1g, 10.5mmoles) were dissolved in warm ethanol (5ml) and c.hydrochloric acid (1 drop) was added. The reaction mixture was allowed to cool to room temperature, and then in ice, and the resulting solid filtered off (0.157g), mp 150-2°.

(b) Diethyl 2-cyano-6-(fluoromethyl)-1,4-dihydro-4-(3-nitrophenyl)-3,5-pyridinedicarboxylate

The product of step (a) (0.45g, 0.8mmoles) was dissolved in 95% aqueous ethanol (20ml) by heating, and then potassium hydroxide (10.16ml of 0.2M in 95% aqueous

ethanol) was added dropwise. The solution was heated at reflux for 3 hours and then the ethanol removed in vacuo. The residue was dissolved in water (75ml), 5% aqueous sodium hydroxide ( 6ml) and chloroform (100ml). The aqueous layer was separated and extracted several times with chloroform. The combined extracts were washed with water, dried ($MgSO_4$) and the solvent was removed in vacuo. The residue was crystallised from 2-propanol to give the title compound (0.19g) mp 147-8$^{\circ}$(dec.).

Example 115

Diethyl 2,6-di-(fluoromethyl)-1,4-dihydro-4-(3-nitrophenyl)-3,5-pyridinedicarboxylate

3-Nitrobenzaldehyde (1.51g, 10mmoles), ethyl 4-fluoro-3-oxo-butanoate (3g, 20mmoles) and aqueous ammonia (1.1ml, d=0.88) in ethanol (15ml) were heated at reflux for 14 hours; more aqueous ammonia (0.55ml) was added after 6 hours. The solvent was removed in vacuo and the residue was chromatographed on silica eluting with ether/petroleum ether (60-80$^{\circ}$) mixtures and the product obtained was recrystallised from 2-propanol to give the title compound as yellow crystals (0.4g) mp 113-4$^{\circ}$.

Example 116

(+) 3-Methyl 5-(1-methylethyl) 4-(2,3-dichlorophenyl)-2-(fluoromethyl)-1,4-dihydro-6-methyl-3,5-pyridinedicarboxylate

Diastereomer A (0.58g, from Example 57) was dissolved in acetonitrile (6ml) and formic acid (0.19ml) and zinc dust (0.6g) were added in turn. The reaction mixture was stirred for 2.5 hours and then cooled in ice while chloroform (20ml) and water (20ml) were added. The aqueous layer was acidified with dilute hydrochloric acid; the organic layer was separated and the aqueous layer re-extracted with chloroform. The combined organic extracts were dried ($Na_2SO_4$) and the solvent removed <u>in vacuo</u> to afford 3-methyl 4-(2,3-dichlorophenyl)-2-(fluoromethyl)-1,4-dihydro-6-methyl-3,5-pyridinedicarboxylate.

The mono-acid was azeotroped once with carbon tetrachloride and then dissolved in ethyl acetate (10ml). 2-Propanol (0.7ml) and dicyclohexylcarbodiimide (1.75g) were added and the mixture stirred at room temperature overnight and then heated at 60$^o$ for 2 hours. The solvent was removed <u>in vacuo</u> and the residue chromatographed on silica eluting with methylene chloride, followed by recrystallisation from hexane, to give the title compound (0.2g) mp 124-5$^o$ $[\alpha]_D^{24.5}$ +38.2$^o$ (c 0.1 in ethanol).

<u>Example 117</u>

<u>(-) 3-Methyl 5-(1-methylethyl) 4-(2,3-dichlorophenyl)-2-(fluoromethyl)-1,4-dihydro-6-methyl-3,5-</u>

pyridinedicarboxylate

Prepared as in Example 116, using diastereomer B from Example 57. Recrystallised from methanol/hexane mp 124-5° $[\alpha]_D^{24}$ -42.3° (c 0.11 in ethanol).

Example 118

(+) 5-Cyclopentyl 3-methyl 4-(2,3-dichlorophenyl)-2-(fluoromethyl)-1,4-dihydro-6-methyl-3,5-pyridinedicarboxylate

Prepared as in Example 116, using diastereomer A from Example 57 and esterifying with cyclopentanol. Recrystallised from hexane mp 89-91°, $[\alpha]_D^{24.5}$ +62.9° (c 0.1 in ethanol).

Example 119

(-) 5-Cyclopentyl 3-methyl 4-(2,3-dichlorophenyl)-2-(fluoromethyl)-1,4-dihydro-6-methyl-3,5-pyridinedicarboxylate

Prepared as in Example 118, using diastereomer B from Example 57 and esterifying with cyclopentanol.

Examples of Intermediates

Example A

Methyl 4-fluoro-3-oxo-butanoate

Fluoroacetyl chloride (7.1g, 73mmoles) was added dropwise to a stirred solution of 2,2-dimethyl-1,3-dioxane-4,6-dione (10.65g, 74mmoles) and pyridine (16.85ml, 210mmoles) in methylene chloride (75ml) keeping the temperature below 10°. After stirring for 16 hours at room temperature the solution was diluted with methylene chloride (100ml) and then washed with 1N hydrochloric acid (200ml) and water (100ml). The organic extract was dried ($Na_2SO_4$) and the solvent removed *in vacuo*. The residue was dissolved in methanol (150ml) and the solution heated at reflux for 2.5 hours. Removal of the solvent followed by distillation at 60-80° (bath temp)/14mm Hg gave methyl 4-fluoro-3-oxobutanoate (6.4g).

The compounds of Examples B and C were prepared using appropriate starting materials and the method of Example A.

Example B

1-Methylethyl 4-fluoro-3-oxobutanoate

Colourless oil, bp 100-120° (bath temp)/12mm Hg.

Example C

Tetrahydro-4H-pyran-4-yl 4-fluoro-3-oxobutanoate

Nmr ($CDCl_3$) $\delta$ 5.0(m,H), 4.9(d,2H,J=48Hz).

Example D

2-Methoxyethyl 4-fluoro-3-oxobutanoate

Ethyl 4-fluoro-3-oxobutanoate (2.1g) was heated at reflux in 2-methoxyethanol (10ml) for 3 hours. The solvent was removed *in vacuo* and the residue distilled to give the title compound as a colourless oil (1.75g). Nmr ($CDCl_3$) $\delta$ 4.9 (d,2H,J-48Hz), 3.4 (s,3H).

The compounds of Examples E and F were prepared using appropriate starting materials and the method of Example D.

Example E

2-Propoxyethyl 4-fluoro-3-oxobutanoate

Nmr ($CDCl_3$) $\delta$ 4.9 (d,2H,J=47Hz), 0.9 (t,3H,J=7Hz).

Example F

2-Phenoxyethyl 4-fluoro-3-oxobutanoate

Nmr ($CDCl_3$) $\delta$ 7.5-6.9 (m,5H), (4.9 d,2H,J=48Hz).

Example G

Methyl 3-amino-4-fluoro-2-butenoate

Ammonia was bubbled through a solution of methyl 4-fluoro-3-oxobutanoate (2.6g) in methanol (26ml) at $0^{\circ}$ for 3 hours. After stirring overnight at room temperature the solvent was removed *in vacuo* and the residue distilled (bp $100^{\circ}$ at 20 mm Hg) to give the title compound (1.3g) Nmr ($CDCl_3$) $\delta$ 4.9 (d,2H,J=48Hz), 4.6 (s,H), (3.7 s,3H).

The compounds of Examples H to J were prepared using appropriate starting materials and the method of Example G.

Example H

Ethyl 3-amino-4-fluoro-2-butenoate

$M^+$ 147; nmr ($D_6$-DMSO) $\delta$ 4.9 (d,2H,J=46Hz), 4.5 (s,H).

Example I

Tetrahydro-4H-pyran-4-yl 3-amino-2-butenoate

Colourless crystals mp 88-90°.

Example J

1-Ethylpropyl 3-amino-2-butenoate

Pale yellow oil, bp 143-8°/12mm Hg.

Example K

(S)-2,2,2-Trichloro-1-phenylethyl 3-oxobutanoate

Diketene (3.7ml, 47mmoles) was added slowly to a stirred mixture of (S)-alpha-(trichloromethyl) phenylmethanol (9.2g, 41mmoles) and triethylamine (0.05ml) kept at 80-90°. The mixture was maintained for 5 hours at 90°. The cooled reaction mixture was purified using HPLC eluting with methylene chloride/petroleum ether 60-80° to give the title compound (11g) as an oil. Nmr ($CDCl_3$) $\delta$ 6.39 (s,H), 3.61 (s,2H), 2.31 (s,3H).

The compound of Example L was obtained by the same method.

Example L

2,2,2-Trichloro-1-phenylethyl 3-oxobutanoate

Colourless solid, nmr ($CDCl_3$) $\delta$ 6.39 (s,H), 3.61

(s,2H), 2.31 (s,3H).

Example M

Tetrahydro-4H-pyran-4-yl 3-oxobutanoate

A solution of tetrahydro-4H-pyran-4-ol (1.6ml, 16.8mmoles) and 5-acetyl-2,2-dimethyl-1,3-dioxane-4,6-dione (3.0g, 16.1mmoles) in dry benzene (50ml) was heated under reflux for 4 hours. The solvent was removed *in vacuo* and the residue distilled at 146-151°/14mm Hg to afford the title product as a colourless oil, 2.84g. Nmr ($CDCl_3$) $\delta$ 5.1 (m,H), 3.5 (s,3H).

The esters of Examples N to R were prepared using appropriate starting materials and the method of Example M.

Example N

1-Ethylpropyl 3-oxobutanoate

Colourless oil, bp 128-38° (bath temp)/14mm Hg.

Example O

1-Methyl-1-phenylethyl 3-oxobutanoate

Colourless oil, bp 108-110° (bath temp)/0.03mm Hg.

Example P

1-Methylcyclopentyl 3-oxobutanoate

Colourless oil, bp 134-145° (bath temp)/14mm Hg.

Example Q

4-(1-Diphenylmethylazetidinyl) 3-oxobutanoate

Pale yellow oil. $M^+$ 323.

Example R

3-Oxetanyl 3-oxobutanoate

Pale yellow oil 165-70° (bath temp)/12mm Hg.

Example S

1-Chloro-4-fluoro-2-(trifluoromethyl)benzene

4-Chloro-3-(trifluoromethyl)benzenamine (19.5g, 100mmoles), water (40ml) and c.hydrochloric acid (40ml) were heated with stirring on a steam bath until a white solid formed. The mixture was cooled (ice-salt bath) and a solution of sodium nitrite (7g, 101mmoles) in water (15ml) was added over 15 mins. After stirring for a further hour at 0°, tetrafluoroboric acid (30g of 40% aqueous solution) was added dropwise over 15 minutes. After one hour the solid was filtered off, washed with water (10ml), methanol (30ml) and ether (30ml) and then dried *in vacuo*. The dry compound was heated at 140°-180° until no more fumes were observed. The cooled residue was dissolved in ethyl acetate, washed with 5% aqueous sodium hydroxide, dried ($Na_2SO_4$) and the solvent was removed *in vacuo*. The residue was distilled *in vacuo* (12mmHg, oven temperature 50°-55°) to give the sub-title compound as a colourless oil (7.5g). $M^+$ 200/198; nmr ($CDCl_3$) $\delta$ 7.8-7.2 (m).

Example T

2-Chloro-5-fluoro-3-(trifluoromethyl)benzaldehyde and 3-Chloro-6-fluoro-2-(trifluoromethyl)benzaldehyde

Butyl lithium (60.4ml of 1.6M in hexane, 97mmoles) was added with stirring over 1.5 hours under nitrogen to a solution of 1-chloro-4-fluoro-2-(trifluoromethyl)benzene (17.8g, 91mmoles) in dry tetrahydrofuran (150ml) at $-73^{o}$. After a further 1.5 hours at this temperature, N-methyl-N-phenylformamide (10.86ml, 90mmoles) in dry tetrahydrofuran (20ml) was added over 0.5 hours. After 15 minutes the reaction mixture was poured onto 10% aqueous sulphuric acid. The ethereal layer was separated, washed with saturated sodium bicarbonate, dried ($Na_2SO_4$) and the solvent evaporated. The residue was purified by HPLC eluting with ethyl acetate/petroleum ether 60-$80^{o}$ mixtures. 2-Chloro-5-fluoro-3-(trifluoromethyl) benzaldehyde (0.5g) was eluted first.

$M^+$ 226/228; nmr ($CDCl_3$) $\delta$ 10.5 (s,H).

Further elution afforded 3-chloro-6-fluoro-2-(trifluoromethyl)benzaldehyde (8.35g).

$M^+$ 226/228; nmr ($CDCl_3$) $\delta$ 10.5 (q,H).

Example U

2-Chloro-3-(trifluoromethyl) benzaldehyde

Butyl lithium (36.4ml of 1.6M in hexane) was added to a stirred solution at $-65^{o}$ of 1-chloro-2-(trifluoromethyl)-benzene (10g) in dry tetrahydrofuran (100ml) over 20 mins. After stirring for 1.5 hours at $-65^{o}$, a solution of N-methyl-N-phenylformamide (6.85ml)

in tetrahydrofuran (30ml) was added over 1 hour. The reaction mixture was left at this temperature for 1.5 hours and then allowed to reach room temperature. It was then poured onto 10% sulphuric acid, extracted with ether and the organic extract was washed with brine, dried ($Na_2SO_4$) and the solvent removed in vacuo. The residue was distilled (20mmHg, oven temperature 100-125°); the distillate was cooled, filtered and the solid washed with petroleum ether (60-80°) to give the desired aldehyde (3.5g) as a colourless solid.
$M^+$ 210/208, nmr ($CDCl_3$) $\delta$ 10.75 (s,H).

Example V

2,3-Dichloro-6-fluorobenzaldehyde

Butyl lithium (48ml of 1.6M in hexane, 52.3mmoles) was added with stirring over 1.5 hours under nitrogen to a solution of 1,2-dichloro-4-fluorobenzene (7.85g, 47.6mmoles) in dry tetrahydrofuran (120ml) at -68°. The solution was stirred at -68° for 2 hours and then N-methyl-N-phenylformamide (6.48ml) in dry tetrahydrofuran (15ml) was added over 1.5 hours. After a further 1.5 hours at -68°, the reaction mixture was poured into 10% aqueous sulphuric acid and ether. The ethereal layer was separated, washed with brine, dried ($Na_2SO_4$) and evaporated to give the desired aldehyde (8g).
$M^+$ 196/194/192, nmr ($CDCl_3$) $\delta$ 10.5 (s,H).

Example W

| | % w/w | Range % w/w |
|---|---|---|
| Compound of formula I | 5 | 1-20 |
| Microcystalline cellulose | 50 | 10-80 |
| Spray dried lactose | 37.75 | 10-80 |
| Magnesium stearate | 1 | 0.25-2 |
| Colloidal silicon dioxide | 0.25 | 0.1-1 |
| Cross linked sodium carboxy methyl cellulose | 3 | 1-5 |
| Hydroxypropylmethylcellulose (coating) | 3 | 1-5 |

This formulation is made up as a direct compression tablet, or without compression or coating, may be filled into a gelatine capsule.

Example X

| | % w/w | Range % w/w |
|---|---|---|
| Compound of formula I | 5 | 1-20 |
| Microcystalline cellulose | 50 | 10-80 |
| Lactose | 35.75 | 10-80 |
| Polyvinylpyrrolidone | 2 | 1-5 |
| Magnesium stearate | 1 | 0.25-2 |
| Colloidal silicon dioxide | 0.25 | 0.1-1 |
| Cross linked sodium carboxy methyl cellulose | 3 | 1-5 |
| Hydroxypropyl methyl cellulose | | |

| | | |
|---|---|---|
| (coating) | 3 | 1-5 |

This formulation is made up as a granulate and then compressed into a tablet. Alternatively the granules may be filled into a gelatine capsule.

0125803

What we claim is:-

1. A compound of formula I,

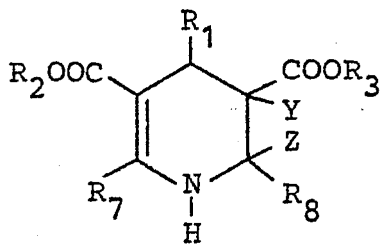

I

in which $R_1$ represents benzofurazanyl, pyridyl or phenyl, the pyridyl or phenyl being substituted by one or more of the groups halogen, nitro, -CN, $-OR_9$ $-S(O)_pR_9$, or alkyl C1 to 6 optionally substituted by halogen,

p is 0, 1 or 2,

$R_2$ and $R_3$, which may be the same or different, each represent hydrogen; alkyl C1 to 6 optionally substituted by one or more of the groups halogen, cyano, $-XR_4$, $-NR_5R_6$ or phenyl; cycloalkyl C3 to 8 optionally substituted by alkyl C1 to 6; a 4, 5 or 6 membered oxygen or nitrogen containing heterocyclic ring which is optionally substituted by alkyl C1 to 6 the alkyl in turn optionally being substituted by one or more phenyl groups;

$R_5$ and $R_6$, which may be the same or different, each represent alkyl C1 to 6 optionally substituted by phenyl,

Y and Z together form a bond, and additionally, when $R_8$ is an electron withdrawing group Y may be hydrogen and Z may be hydroxy,

one of $R_7$ and $R_8$ represents alkyl C1 to 6 and the other represents $-CONR_{10}R_{11}$; $-CSNH_2$; $-C(=NH)SR_9$; $-S(O)_mR_9$; phenyl optionally substituted by one or more of alkyl C1 to 6, halogen, alkoxy C1 to 6 or nitro; alkyl C1 to 6 substituted by halogen; or furanyl,

or $R_7$ and $R_8$ may be the same or different and each represents phenyl optionally substituted by one or more of alkyl C1 to 6, halogen, alkoxy C1 to 6 or nitro; amino; alkyl C1 to 6 substituted by halogen; -CN; $-CH_2OH$; -CHO or $-CH(OR_9)_2$,

X is O or S,

m is 0 or 1,

$R_4$ is alkyl C1 to 6 or phenyl,

$R_9$ is alkyl C1 to 6,

$R_{10}$ and $R_{11}$ each independently represent hydrogen or alkyl C1 to 6, or together with the nitrogen atom to which they are attached form a 5 or 6 membered heterocyclic ring,

provided that A) when $R_7$ is alkyl C1 to 6, Y and Z together form a bond, and

i) $R_1$ represents benzofurazanoyl then $R_8$ does not represent $-CF_3$, or

0125803

ii) when $R_1$ represents 2-nitrophenyl, or 2-chlorophenyl and $R_2$ and $R_3$ are both ethyl, then $R_8$ does not represent mono-chloromethyl,

iii) when $R_1$ represents 3-nitrophenyl and $R_2$ and $R_3$ are both ethyl, then $R_8$ does not represent unsubstituted phenyl,

B) when neither of $R_7$ and $R_8$ is alkyl C1 to 6, Y and Z together form a bond and

iv) $R_2$ and $R_3$ are both ethyl then $R_7$ and $R_8$ are not both $-CF_3$, or

v) one of $R_7$ or $R_8$ is amino then the other is not phenyl or amino, or

vi) one of $R_7$ or $R_8$ is -CN, $-CH_2OH$, -CHO or $-CH(OR_9)_2$ then the other is not -CN, $-CH_2OH$, -CHO or $-CH(OR_9)_2$, and

C) both of $R_7$ and $R_8$ are not optionally substituted phenyl,

and pharmaceutically acceptable acid addition salts of those compounds containing a basic nitrogen atom.

2. A compound according to Claim 1, wherein

$R_1$ is nitrophenyl; (trifluoromethyl)phenyl; mono- or poly-fluorophenyl; mono- or poly-chlorophenyl; chloro- and/or fluoro-(trifluoromethyl)phenyl; (alkylthio)pyridyl; alkyl- and/or chloro- and/or alkoxy-nitrophenyl; mixed chloro- and fluoro-phenyl; mono- or poly- alkoxy-phenyl;

alkylphenyl; (alkylthio)phenyl; (alkylsulphonyl)phenyl, or 4-benzofurazanyl,

$R_2$ and $R_3$ are selected from alkyl C1 to 4; 2-alkoxy C1 to 3 - ethyl; 2-phenoxy- ethyl; cycloalkyl C4 to 6 optionally substituted by methyl; an oxetanyl, azetidinyl, piperidinyl or tetrahydropyranyl ring optionally substituted by phenylmethyl or diphenylmethyl; alkyl C1 to 4 - (phenylmethyl)aminoethyl; cyano- or alkyl C1 to 4 - thio- alkyl C1 to 4; phenyl alkyl C1 to 4 or $-CH(C_6H_5)CCl_3$,

$R_7$ is methyl, and

$R_8$ is chloro- or fluoro- alkyl C1 or 2, $-CSNH_2$, -CON(alkyl C 1 to 4)$_2$, -COmorpholino, -COimidazolyl, -C(=NH)S-alkyl C1 to 4, -S-alkyl C1 to 4, -S(O)-alkyl C1 to 4, or phenyl substituted by one or two chlorine, nitro, methoxy or methyl groups.

3. A compound according to Claim 1, wherein $R_1$ is phenyl carrying a 2-nitro or a 2-$CF_3$ group or at least two substituents selected from chloro, fluoro, alkyl C1 to 6, $-CF_3$ and nitro; $R_2$ is alkyl C1 to 6, or is oxetan -3-yl, $R_3$ is alkyl C1 to 6, $R_7$ is alkyl C1 to 6, $R_8$ is fluoromethyl, and Y and Z together form a bond.

4. A compound according to Claim 1, wherein $R_1$ is phenyl carrying at least two substituents selected from chloro, fluoro, $-CF_3$, methyl and nitro, $R_3$ and $R_7$ are both methyl, $R_8$ is $-CH_2F$, $R_2$ is isopropyl or

cyclopentyl and Y and Z together form a bond.

5. A compound according to Claim 1, wherein $R_1$ represents benzofurazanyl, pyridyl or phenyl, the pyridyl or phenyl being substituted by one or more of the groups halogen, nitro, trihalomethyl or $-SR_9$; $R_2$ and $R_3$ each represent alkyl C1 to 6, $-(CH_2)_n R_4$, $-(CH_2)_nCN$, $-CH(C_6H_5)CCl_3$ or $-(CH_2)_n NR_5R_6$; Y and Z together form a bond; one of $R_7$ and $R_8$ represents alkyl C1 to 6 and the other represents $-CONR_{10}R_{11}$; $-CSNH_2$; $-C(=NH)SR_9$; $-S(O)_mR_9$; phenyl substituted by one or more of alkyl C1 to 6, halogen, alkoxy C1 to 6 or nitro; or alkyl C1 to 6 substituted by halogen; $R_4$ and $R_9$ are each alkyl C1 to 6; $R_{10}$ and $R_{11}$ each represent hydrogen or alkyl C1 to 6, n is 2, 3 or 4 and provisos i) and ii) apply.

6. 3-Methyl 5-(1-methylethyl) 4-(3-chloro-6-fluoro-2-(trifluoromethyl)phenyl)-2-(fluoromethyl)-1,4-dihydro-6-methyl-3,5-pyridinedicarboxylate.

7. 3-Methyl 5-(1-methylethyl) 4-(2,3-dichlorophenyl)-2--(fluoromethyl)-1,4-dihydro-6-methyl-3,5-pyridinedicarboxylate,

3-Methyl 5-(1-methylethyl) 2-(fluoromethyl)-1,4-dihydro-6-methyl-4-(2-methyl-3-nitrophenyl)-3,5-pyridinedicarboxylate,

5-Cyclopentyl 3-methyl 4-(2,3-dichlorophenyl)-2-

(fluoromethyl)-1,4-dihydro-6-methyl-3,5-pyridinedicarboxylate,

3-Methyl 5-(1-methylethyl) 4-(2-chloro-3-nitrophenyl)-2-(fluoromethyl)-1,4-dihydro-6-methyl-3,5-pyridinedicarboxylate,

3-Methyl 5-(1-methylethyl) 2-(fluoromethyl)-4-(2-fluoro-6-(trifluoromethyl)phenyl)-1,4-dihydro-6-methyl-3,5-pyridinedicarboxylate,

3-Methyl 5-(1-methylethyl) 4-(2,3-dichloro-6-fluorophenyl)-2-(fluoromethyl)-1,4-dihydro-6-methyl-3,5-pyridinedicarboxylate,

5-Ethyl 3-methyl 2-(fluoromethyl)-1,4-dihydro-6-methyl-4-(3-nitrophenyl)-3,5-pyridinedicarboxylate,

3-Methyl 5-(1-methylethyl) 2-(fluoromethyl)-1,4-dihydro-6-methyl-4-(3-nitrophenyl)-3,5-pyridinedicarboxylate,

3-Methyl 5-(2-methylpropyl) 2-(fluoromethyl)-1,4-dihydro-6-methyl-4-(3-nitrophenyl)-3,5-pyridinedicarboxylate,

3-Ethyl 5-methyl 2-(fluoromethyl)-1,4-dihydro-6-methyl-4-(3-nitrophenyl)-3,5-pyridinedicarboxylate,

Diethyl 2-(fluoromethyl)-1,4-dihydro-6-methyl-4-(2-(trifluoromethyl)phenyl)-3,5-pyridinedicarboxylate,

Diethyl 4-(4-benzofurazanyl)-2-(fluormethyl)-1,4-dihydro-6-methyl-3,5-pyridinedicarboxylate,

Dimethyl 2-(fluoromethyl)-1,4-dihydro-6-methyl-4--(2,3,4,5,6-pentafluorophenyl)-3,5-pyridinedicarboxylate,

5-Methyl 3-(1-methylethyl) 2-(fluoromethyl)-1,4-dihydro-6-methyl-4-(3-nitrophenyl)-3,5-pyridinedicarboxylate,

5-Ethyl 3-methyl 4-(2,3-dichlorophenyl)-2-(fluoromethyl)-1,4-dihydro-6-methyl-3,5-pyridinedicarboxylate,

Diethyl 2-(fluoromethyl)-1,4-dihydro-6-methyl-4-(2-(methylthio)-3-pyridyl)-3,5-pyridinedicarboxylate,

3-Methyl 5-(2-(methyl(phenylmethyl)amino)ethyl) 2-(fluoromethyl)-1,4-dihydro-6-methyl-4-(3-nitrophenyl)-3,5-pyridinedicarboxylate oxalate,

5-(2-Methoxyethyl) 3-(1-methylethyl) 4-(2,3-dichlorophenyl)-2-(fluoromethyl)-1,4-dihydro-6-methyl-3,5-pyridinedicarboxylate,

5-(2-Cyanoethyl) 3-(1-methylethyl) 2-(fluoromethyl)-1,4-dihydro-6-methyl-4-(3-nitrophenyl)-3,5-pyridinedicarboxylate,

3-(1-Methylethyl) 5-(2-(methylthio)ethyl) 2-(fluoromethyl)-1,4-dihydro-6-methyl-4-(3-nitrophenyl)-3,5-pyridinedicarboxylate,

3-Methyl 5-(1-methylethyl) 4-(2-chloro-5-nitrophenyl)-2-(fluoromethyl)-1,4-dihydro-6-methyl-3,5-pyridinedicarboxylate,

3-Ethyl 5-methyl 4-(2,3-dichlorophenyl)-2-(fluoromethyl)-1,4-dihydro-6-methyl-3,5-pyridinedicarboxylate,

3-Methyl 5-(1-methylethyl) 2-(fluoromethyl)-1,4-dihydro-6-methyl-4-(2-methyl-5-nitrophenyl)-3,5-pyridinedicarboxylate,

3-(2-Methoxyethyl) 5-(1-methylethyl) 2-(fluoromethyl)-1,4-dihydro-6-methyl-4-(3-nitrophenyl)-3,5-pyridinedicarboxylate,

5-(2-Methoxyethyl) 3-(1-methylethyl) 2-(fluoromethyl)-1,4-dihydro-6-methyl-4-(3-nitrophenyl)-3,5-pyridinedicarboxylate,

3-Methyl 5-(1-methylethyl) 4-(2-chloro-3-(tri-fluoromethyl)phenyl)-2-(fluoromethyl)-1,4-dihydro-6-methyl-3,5-pyridinedicarboxylate,

5-(1-Methylethyl) 3-(tetrahydro-4H-pyran-4-yl) 2-(fluoromethyl)-1,4-dihydro-6-methyl-4-(3-nitrophenyl)-3,5-pyridinedicarboxylate,

5-(1-Methylethyl) 3-(2-phenoxyethyl) 2-(fluoromethyl)-1,4-dihydro-6-methyl-4-(3-nitrophenyl)-3,5-pyridinedicarboxylate,

5-Methyl 3-(tetrahydro-4H-pyran-4-yl) 2-(fluoromethyl) 1,4-dihydro-6-methyl-4-(3-nitrophenyl)-3,5-pyridinedicarboxylate,

5-Cyclohexyl 3-methyl 4-(2,3-dichlorophenyl)-2-

(fluoromethyl)-1,4-dihydro-6-methyl-3,5-pyridinedicarboxylate,

5-Cyclopentyl 3-methyl 2-(fluoromethyl)-1,4-dihydro-6-methyl-4-(2-methyl-3-nitrophenyl)-3,5-pyridinedicarboxylate,

3-Methyl 5-(1-methylethyl) 4-(2,3-dimethoxyphenyl)-2-(fluoromethyl)-1,4-dihydro-6-methyl-3,5-pyridinedicarboxylate,

3-Methyl 5-(tetrahydro-4H-pyran-4-yl) 4-(2,3-dichlorophenyl)-2-(fluoromethyl)-1,4-dihydro-6-methyl-3,5-pyridinedicarboxylate,

5-Cyclopentyl 3-methyl 2-(fluoromethyl)-1,4-dihydro-6-methyl-4-(2-(trifluoromethyl)phenyl)-3,5-pyridinedicarboxylate,

5-Cyclopentyl 3-methyl 4-(3-chloro-6-fluoro-2-(trifluoromethyl)phenyl)-2-(fluoromethyl)-1,4-dihydro-6-methyl-3,5-pyridinedicarboxylate,

5-(1-Ethylpropyl) 3-methyl 4-(2,3-dichlorophenyl)-2-(fluoromethyl)-1,4-dihydro-6-methyl-3,5-pyridinedicarboxylate,

3-Methyl 5-(1-methylethyl) 2-(fluoromethyl)-1,4-dihydro-4-(2-methoxy-3-nitrophenyl)-6-methyl-3,5-pyridinedicarboxylate,

3-Methyl 5-(1-methylethyl) 2-(fluoromethyl)-1,4-dihydro-6-methyl-4-(2-(trifluoromethyl)phenyl)-3,5-pyridinedicarboxylate,

3-Methyl 5-(1-methylethyl) 2-(fluoromethyl)-1,4-dihydro-6-methyl-4-(2-nitrophenyl)-3,5-pyridinedicarboxylate,

3-Methyl 5-(1-methylethyl) 2-(fluoromethyl)-4-(2-fluorophenyl)-1,4-dihydro-6-methyl-3,5-pyridinedicarboxylate,

3-Methyl 5-(1-methylethyl) 2-(fluoromethyl)-1,4-dihydro-6-methyl-4-(2-methylphenyl)-3,5-pyridinedicarboxylate,

3-Methyl 5-(1-methylethyl) 4-(2-chlorophenyl)-2-(fluoromethyl)-1,4-dihydro-6-methyl-3,5-pyridinecarboxylate,

3-Methyl 5-(1-methylethyl) 2-(fluoromethyl)-1,4-dihydro-6-methyl-4-(2-(methylthio)phenyl)-3,5-pyridinedicarboxylate,

3-Methyl 5-(1-methylethyl) 2-(fluoromethyl)-1,4-dihydro-6-methyl-4-(2-(methylsulphonyl)phenyl)-3,5-pyridinedicarboxylate,

3-Methyl 5-(1-methylethyl) 4-(3-chloro-2-methylphenyl)-2-(fluoromethyl)-1,4-dihydro-6-methyl-3,5-pyridinedicarboxylate,

3-Methyl 5-(1-methylethyl) 4-(2,3-dimethylphenyl)-2-(fluoromethyl)-1,4-dihydro-6-methyl-3,5-pyridinedicarboxylate,

3-Methyl 5-(1-methylethyl) 4-(3-cyanophenyl)-2-(fluoromethyl)-1,4-dihydro-6-methyl-3,5-pyridinedicarboxylate,

3-Methyl 5-(1-methylethyl) 4-(3-chlorophenyl)-2-(fluoromethyl)-1,4-dihydro-6-methyl-3,5-pyridinedicarboxylate,

Diethyl 1,2,3,4-tetrahydro-2-hydroxy-6-methyl-4-(3-nitrophenyl)-2-(trifluoromethyl)-3,5-pyridinedicarboxylate,

3-Ethyl 5-methyl 4-(2,3-dichlorophenyl)-1,2,3,4-tetrahydro-2-hydroxy-6-methyl-2-(trifluoromethyl)-3,5-pyridinedicarboxylate,

Diethyl 1,2,3,4-tetrahydro-2-hydroxy-6-methyl-4-(3-nitrophenyl)-2-(pentafluoroethyl)-3,5-pyridinedicarboxylate,

5-Cyclobutyl 3-methyl 4-(2,3-dichlorophenyl)-2-(fluoromethyl)-1,4-dihydro-6-methyl-3,5-pyridinedicarboxylate,

3-Methyl 5-(3-oxetanyl) 4-(2,3-dichlorophenyl)-2-(fluoromethyl)-1,4-dihydro-6-methyl-3,5-pyridinedicarboxylate,

Diethyl 1,2,3,4-tetrahydro-2-hydroxy-6-methyl-4-(3-nitrophenyl)-2-(trichloromethyl)-3,5-pyridinedicarboxylate,

3-Methyl 5-((S)-2,2,2-trichloro-1-phenylethyl) 4-(2,3-dichlorophenyl)-2-(fluoromethyl)-1,4-dihydro-6-methyl-3,5-pyridinedicarboxylate,

3-Methyl 5-(2,2,2-trichloro-1-phenylethyl) 4-(2,3-dichlorophenyl)-2-(fluoromethyl)-1,4-dihydro-6-methyl-3,5-pyridinedicarboxylate,

Diethyl 2-(difluoromethyl)-1,4-dihydro-6-methyl-4-(3-nitrophenyl)-3,5-pyridinedicarboxylate,

Diethyl 1,4-dihydro-2-methyl-6-methylthio-4-(3-nitrophenyl)-3,5-pyridinedicarboxylate,

Diethyl 1,4-dihydro-2-(4-methoxyphenyl)-6-methyl-4-(3-nitrophenyl)-3,5-pyridinedicarboxylate,

Diethyl 2-(dichloromethyl)-1,4-dihydro-6-methyl-4-(3-nitrophenyl)-3,5-pyridinedicarboxylate,

Diethyl 1,4-dihydro-2-methyl-6-phenyl-4-(2-(trifluoromethyl)phenyl)-3,5-pyridinedicarboxylate,

5-Methyl 3-(1-methylethyl) 4-(4-benzofurazanyl)-1,4-dihydro-2-methyl-6-phenyl-3,5-pyridinedicarboxylate,

5-Methyl 3-(1-methylethyl) 1,4-dihydro-2-methyl-6-phenyl-4-(2-(trifluoromethyl)phenyl)-3,5-pyridinedicarboxylate,

5-Ethyl 3-methyl 4-(2,3-dichlorophenyl)-1,2,3,4-tetrahydro-2-hydroxy-6-methyl-2-phenyl-3,5-pyridinedicarboxylate,

Diethyl 4-(4-benzofurazanyl)-2-diethoxymethyl-1,4,5,6-tetrahydro-6-hydroxy-6-(triflurommethyl)-3,5-

pyridinedicarboxylate,

5-(1-(Diphenylmethyl)-3-azetidinyl) 3-methyl 4-(2,3-dichlorophenyl)-2-(fluoromethyl)-1,4-dihydro-6-methyl-3,5-pyridinedicarboxylate,

3-Methyl 5-(1-(phenylmethyl)-4-piperidinyl) 4-(2,3-dichlorophenyl)-2-(fluoromethyl)-1,4-dihydro-6-methyl-3,5-pyridinedicarboxylate,

5-(1,1-Dimethylethyl) 3-methyl 4-(2,3-dichlorophenyl) -2-(fluoromethyl)-1,4-dihydro-6-methyl-3,5-pyridinedicarboxylate,

3-Methyl 5-(1-methyl-1-phenylethyl) 4-(2,3-dichlorophenyl)-2-(fluoromethyl)-1,4-dihydro-6-methyl-3,5-pyridinedicarboxylate,

3-Methyl 5-(1-methylcyclopentyl) 4-(2,3-dichlorophenyl)-2-(fluoromethyl)-1,4-dihydro-6-methyl-3,5-pyridinedicarboxylate,

3-Methyl 5-(2,2,2-trichloro-1-phenylethyl) 2-(fluoromethyl)-1,4-dihydro-6-methyl-4-(3-nitrophenyl) -3,5-pyridinedicarboxylate,

Diethyl 2-(fluoromethyl)-6-formyl-1,4-dihydro-4-(3-nitrophenyl)-3,5-pyridinedicarboxylate,

3-Methyl 5-(1-methylethyl) 4-(4-benzofurazanyl)-1,2,3,4-tetrahydro-2-hydroxy-6-methyl-2-phenyl-3,5-pyridinedicarboxylate,

Diethyl 2-amino-6-(fluoromethyl)-1,4-dihydro-4-

(3-nitrophenyl)-3,5-pyridinedicarboxylate,

Diethyl 2-(fluoromethyl)-1,4,5,6-tetrahydro-6-hydroxy-4-(3-nitrophenyl)-6-phenyl-3,5-pyridinedicarboxylate,

Diethyl 2-(fluoromethyl)-1,4-dihydro-4-(3-nitrophenyl)-6-phenyl-3,5-pyridinedicarboxylate,

3-Ethyl 5-methyl 4-(2,3-dichlorophenyl)-1,4-dihydro-2-methyl-6-phenyl-3,5-pyridinedicarboxylate,

Diethyl 1,4-dihydro-2-methyl-4-(3-nitrophenyl)-6-(4-nitrophenyl)-3,5-pyridinedicarboxylate,

Diethyl 2-(3,4-dichlorophenyl)-1,4-dihydro-6-methyl-4-(3-nitrophenyl)-3,5-pyridinedicarboxylate,

Diethyl 2-(4-chlorophenyl)-1,4-dihydro-6-methyl-4-(3-nitrophenyl)-3,5-pyridinedicarboxylate,

Diethyl 1,4-dihydro-2-methyl-4-(3-nitrophenyl)-6-(trifluoromethyl)-3,5-pyridinedicarboxylate,

Diethyl 2-(3-chlorophenyl)-1,4-dihydro-6-methyl-4-(3-nitrophenyl)-3,5-pyridinedicarboxylate,

Diethyl 1,4-dihydro-2-methyl-4-(3-nitrophenyl)-6-(pentafluoroethyl)-3,5-pyridinedicarboxylate,

5-Ethyl 3-methyl 4-(2,3-dichlorophenyl)-1,4-dihydro-2-methyl-6-(trifluoromethyl)-3,5-pyridinedicarboxylate,

Diethyl 4-(4-benzofurazanyl)-2-(diethoxymethyl)-1,4-dihydro-6-(trifluoromethyl)-3,5-pyridinedicarboxylate,

Diethyl 1,4-dihydro-2-methyl-4-(3-nitrophenyl)-6-(trichloromethyl)-3,5-pyridinedicarboxylate,

Diethyl 1,4-dihydro-2-methyl-6-(methylsulphinyl)-4-(3-nitrophenyl)-3,5-pyridinedicarboxylate,

Diethyl 2-aminocarbonyl-1,4-dihydro-6-methyl-4-(3-nitrophenyl)-3,5-pyridinedicarboxylate,

Diethyl 2-(dimethylaminocarbonyl)-1,4-dihydro-6-methyl-4-(3-nitrophenyl)-3,5-pyridinedicarboxylate,

Diethyl 1,4-dihydro-2-(1H-imidazol-1-ylcarbonyl)-6-methyl-4-(3-nitrophenyl)-3,5-pyridinedicarboxylate,

Diethyl 1,4-dihydro-2-methyl-6-(4-morpholinylcarbonyl)-4-(3-nitrophenyl)-3,5-pyridinedicarboxylate,

Diethyl 2-(aminothioxomethyl)-1,4-dihydro-6-methyl-4-(3-nitrophenyl)-3,5-pyridinedicarboxylate,

Diethyl 1,4-dihydro-2-(imino(methylthio)methyl)-6-methyl-4-(3-nitrophenyl)-3,5-pyridinedicarboxylate hydroiodide,

Diethyl 2-(fluoromethyl)-1,4-dihydro-6-methyl-4-(3-nitrophenyl)-3,5-pyridinedicarboxylate,

Di-(2-propoxyethyl) 2-(fluoromethyl)-1,4-dihydro-6-methyl-4-(3-nitrophenyl)-3,5-pyridinedicarboxylate,

Diethyl 1,2,3,4-tetrahydro-2-hydroxy-6-methyl-4-(3-nitrophenyl)-2-(4-nitrophenyl)-3,5-pyridinedicarboxylate,

5-Methyl 3-(1-methylethyl) 1,4-dihydro-2-methyl-4-(3-nitrophenyl)-6-phenyl-3,5-pyridinedicarboxylate,

Diethyl 1,2,3,4-tetrahydro-2-hydroxy-6-methyl-4-(3-nitrophenyl)-2-phenyl-3,5-pyridinedicarboxylate,

Diethyl 2-(3,4-dichlorophenyl)-1,2,3,4-tetrahydro-2-hydroxy-6-methyl-4-(3-nitrophenyl)-3,5-pyridinedicarboxylate,

Diethyl 2-(4-chlorophenyl)-1,2,3,4-tetrahydro-2-hydroxy-6-methyl-4-(3-nitrophenyl)-3,5-pyridinedicarboxylate,

Diethyl 1,4-dihydro-2-methyl-6-(4-methylphenyl)-4-(3-nitrophenyl)-3,5-pyridinedicarboxylate,

3-Methyl 5-(1-methylethyl) 1,2,3,4-tetrahydro-2-hydroxy-6-methyl-4-(3-nitrophenyl)-2-phenyl-3,5-pyridinedicarboxylate,

Diethyl 2-(3-chlorophenyl)-1,2,3,4-tetrahydro-2-hydroxy-6-methyl-4-(3-nitrophenyl)-3,5-pyridinedicarboxylate,

Diethyl 2-(2-furanyl)-1,4-dihydro-6-methyl-4-(3-nitrophenyl)-3,5-pyridinedicarboxylate,

3-Methyl 5-(1-methylethyl) 2-(difluoromethyl)-1,4-dihydro-6-methyl-4-(3-nitrophenyl)-3,5-pyridinedicarboxylate,

5-Cyclopentyl 3-methyl 2-(difluoromethyl)-1,4-dihydro-6-methyl-4-(2-methyl-3-nitrophenyl)-3,5-pyridinedicarboxylate,

3-Methyl 5-(1-methylethyl) 4-(2,3-dichlorophenyl)-2-(difluoromethyl)-1,4-dihydro-6-methyl-3,5-pyridinedicarboxylate,

Diethyl 2-(fluoromethyl)-1,4-dihydro-6-methyl-4-(3-nitrophenyl)-3,5-pyridinedicarboxylate,

3-(2-Methoxyethyl) 5-(1-methylethyl) 4-(2,3-dichlorophenyl)-2-(difluoromethyl)-1,4-dihydro-6-methyl-3,5-pyridinedicarboxylate,

3-(2-Methoxyethyl) 5-(1-methylethyl) 2-(difluoromethyl)-1,4-dihydro-6-methyl-4-(3-nitrophenyl)-3,5-pyridinedicarboxylate,

Diethyl 4-(4-benzofurazanyl)-2-formyl-1,4-dihydro-6-(trifluoromethyl)-3,5-pyridinedicarboxylate,

Diethyl 4-(4-benzofurazanyl)-1,4-dihydro-2-(hydroxymethyl)-6-trifluoromethyl-3,5-pyridinedicarboxylate,

Diethyl 2-(fluoromethyl)-1,4-dihydro-6-hydroxymethyl-4-(3-nitrophenyl)-3,5-pyridinedicarboxylate,

Diethyl 2-cyano-6-(fluoromethyl)-1,4-dihydro-4-(3-nitrophenyl)-3,5-pyridinedicarboxylate,

Diethyl 2,6-di-(fluoromethyl)-1,4-dihydro-4-(3-nitrophenyl)-3,5-pyridinedicarboxylate,

(+) 3-Methyl 5-(1-methylethyl) 4-(2,3-dichlorophenyl)-2-(fluoromethyl)-1,4-dihydro-6-methyl-3,5-pyridinedicarboxylate,

(-) 3-Methyl 5-(1-methylethyl) 4-(2,3-dichlorophenyl)-2-(fluoromethyl)-1,4-dihydro-6-methyl-3,5-pyridinedicarboxylate,

(+) 5-Cyclopentyl 3-methyl 4-(2,3-dichlorophenyl)-2-(fluoromethyl)-1,4-dihydro-6-methyl-3,5-pyridinedicarboxylate, or

(-) 5-Cyclopentyl 3-methyl 4-(2,3-dichlorophenyl)-2-(fluoromethyl)-1,4-dihydro-6-methyl-3,5-pyridinedicarboxylate.

8. A pharmaceutical formulation containing a compound according to any one of the preceding claims in admixture with a pharmaceutically acceptable adjuvant, diluent or carrier.

9. The use of a compound according to Claim 1 without proviso ii) as a pharmaceutical.

10. A process for the production of a compound of formula I, as defined in Claim 1, or a pharmaceutically acceptable acid addition salt thereof, which comprises

a) reaction of a compound of formula II,

$R_1CHO$ II

with compounds of formulae III and IV,

$R_2OOCCH{=}C(R_7)NH_2$ III

$R_3OOCCH_2COR_8$ IV

in which formulae $R_1$, $R_2$, $R_3$, $R_7$ and $R_8$ are as defined in Claim 1,

b) reaction of a compound of formula V,

$R_1CH{=}C(COOR_3)COR_8$ V

in which $R_1$, $R_3$ and $R_8$ are as defined in Claim 1,

with a compound of formula III,

c) production of a compound of formula I in which Y and Z together form a bond by dehydration of a compound of formula VII,

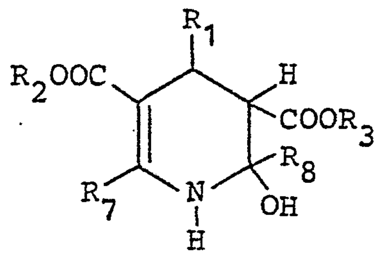

VII

in which $R_1$, $R_2$, $R_3$, $R_7$ and $R_8$ are as defined in Claim 1,

d) production of a compound of formula I in which m is 1 or p is 1 or 2 by selective oxidation of a corresponding compound of formula I in which m is 0, or p is 0 or 1 respectively,

e) production of a compound of formula I in which one of $R_7$ and $R_8$ is $-CONR_{10}R_{11}$ by reaction of an acid halide, imidazole or a mixed anhydride of a corresponding compound of formula I in which one of $R_7$ and $R_8$ is $-COOH$ with a compound $HNR_{10}R_{11}$ in which $R_{10}$ and $R_{11}$ are as defined in Claim 1, or, when the group $-NR_{10}R_{11}$ in the product represents an imidazole, reacting the free carboxylic acid of formula I with N,N'-carbonyldiimidazole,

f) production of a compound of formula I in which one of $R_7$ and $R_8$ is $-CSNH_2$ by reaction of a corresponding compound of formula I in which one of $R_7$ and $R_8$ is -CN with hydrogen sulphide,

g) isomerising a 3,4-dihydropyridine to a corresponding compound of formula I,

h) production of a compound of formula I in which one of $R_7$ and $R_8$ is $-C(=NH)SR_9$ by reaction of a corresponding compound of formula I in which one of $R_7$ and $R_8$ is $-CSNH_2$ with a compound $R_9$-hal, in which $R_9$ is as defined in Claim 1 and hal is a halogen atom,

i) reaction of a compound of formula IV with ammonia and a compound of formula VI,

$$R_1CH{=}C(COOR_2)COR_7 \qquad VI$$

or reaction of a compound of formula V with ammonia and a compound of formula VII,

$$R_2OOCCH_2COR_7 \qquad VII$$

or reaction of compounds of formulae II, IV and VII with ammonia,

in which formulae $R_1$, $R_2$, $R_3$, $R_7$ and $R_8$ are as defined in Claim 1,

j) production of a compound of formula I in which Y and Z together form a bond and one or both of $R_7$ and $R_8$ is $-CHF_2$ or $-CH_2F$ by reaction of a corresponding compound of formula I in which Y and Z together form a bond and one

or both of $R_7$ and $R_8$ is -CHO or $-CH_2L$, where L is -OH or a good leaving group, respectively with a fluorinating agent,

k) production of a compound of formula I in which one of $R_7$ and $R_8$ is -CHO by selective hydrolysis of a corresponding compound of formula I in which one of $R_7$ and $R_8$ is $-CH(OR_9)_2$,

l) production of a compound of formula I in which one of $R_7$ and $R_8$ is $-CH_2OH$ by selective reduction of a corresponding compound of formula I in which one of $R_7$ and $R_8$ is -CHO,

m) production of a compound of formula I in which one of $R_7$ and $R_8$ is -CN by elimination of ROH from a corresponding compound of formula I in which one of $R_7$ and $R_8$ is -CH=NOR, and -OR is a good leaving group,

n) production of a compound of formula I in which at least one of $R_2$ and $R_3$ is hydrogen by reductive cleavage or hydrolysis of a corresponding compound of formula I in which at least one of $R_2$ and $R_3$ is other than hydrogen,

o) production of a compound of formula I in which at least one of $R_2$ and $R_3$ is other than hydrogen by esterification or transesterification of a corresponding compound of formula I in which at least one of $R_2$ and $R_3$ is hydrogen or is a group $R_2$ or $R_3$ other than

that desired in the end product, or

p) production of an optical isomer of a compound of formula I by resolution of a mixture of optical isomers of the compound,

and where desired or necessary converting the resulting compound of formula I to a pharmaceutically acceptable acid addition salt thereof or vice versa.

11. A compound of formula VII as defined in Claim 1.

12. A compound $R_1CHO$ in which $R_1$ is 2-chloro-3-trifluoromethyl phenyl or phenyl substituted by three substituents selected from chloro-, fluoro- and $-CF_3$.

13. 3-Chloro-6-fluoro-2-(trifluoromethyl)benzaldehyde.

What we claim is:-

1. A process for the production of a compound of formula I,

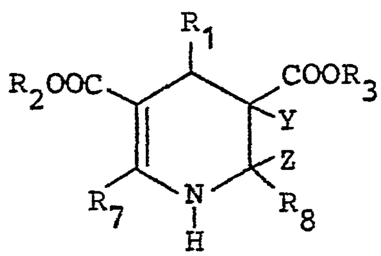

I

in which $R_1$ represents benzofurazanyl, pyridyl or phenyl, the pyridyl or phenyl being substituted by one or more of the groups halogen, nitro, -CN, $-OR_9$, $-S(O)_pR_9$, or alkyl C1 to 6 optionally substituted by halogen,

p is 0, 1 or 2,

$R_2$ and $R_3$, which may be the same or different, each represent hydrogen; alkyl C1 to 6 optionally substituted by one or more of the groups halogen, cyano, $-XR_4$, $-NR_5R_6$ or phenyl; cycloalkyl C3 to 8 optionally substituted by alkyl C1 to 6; a 4, 5 or 6 membered oxygen or nitrogen containing heterocyclic ring which is optionally substituted by alkyl C1 to 6 the alkyl in turn optionally being substituted by one or more phenyl groups;

$R_5$ and $R_6$, which may be the same or different, each represent alkyl C1 to 6 optionally substituted by

phenyl,

Y and Z together form a bond, and additionally, when $R_8$ is an electron withdrawing group Y may be hydrogen and Z may be hydroxy,

one of $R_7$ and $R_8$ represents alkyl C1 to 6 and the other represents $-CONR_{10}R_{11}$; $-CSNH_2$; $-C(=NH)SR_9$; $-S(O)_mR_9$; phenyl optionally substituted by one or more of alkyl C1 to 6, halogen, alkoxy C1 to 6 or nitro; alkyl C1 to 6 substituted by halogen; or furanyl,

or $R_7$ and $R_8$ may be the same or different and each represents phenyl optionally substituted by one or more of alkyl C1 to 6, halogen, alkoxy C1 to 6 or nitro; amino; alkyl C1 to 6 substituted by halogen; -CN; $-CH_2OH$; -CHO or $-CH(OR_9)_2$,

X is O or S,

m is 0 or 1,

$R_4$ is alkyl C1 to 6 or phenyl,

$R_9$ is alkyl C1 to 6,

$R_{10}$ and $R_{11}$ each independently represent hydrogen or alkyl C1 to 6, or together with the nitrogen atom to which they are attached form a 5 or 6 membered heterocyclic ring,

provided that A) when $R_7$ is alkyl C1 to 6, Y and Z together form a bond, and

i) $R_1$ represents benzofurazanoyl then $R_8$ does

0125803

not represent $-CF_3$, or

ii) when $R_1$ represents 2-nitrophenyl, or 2-chlorophenyl and $R_2$ and $R_3$ are both ethyl, then $R_8$ does not represent mono-chloromethyl,

iii) when $R_1$ represents 3-nitrophenyl and $R_2$ and $R_3$ are both ethyl, then $R_8$ does not represent unsubstituted phenyl,

B) when neither of $R_7$ and $R_8$ is alkyl Cl to 6, Y and Z together form a bond and

iv) $R_2$ and $R_3$ are both ethyl then $R_7$ and $R_8$ are not both $-CF_3$, or

v) one of $R_7$ or $R_8$ is amino then the other is not phenyl or amino, or

vi) one of $R_7$ or $R_8$ is -CN, $-CH_2OH$, -CHO or $-CH(OR_9)_2$ then the other is not -CN, $-CH_2OH$, -CHO or $-CH(OR_9)_2$, and

C) both of $R_7$ and $R_8$ are not optionally substituted phenyl,

or a pharmaceutically acceptable acid addition salt of those compounds containing a basic nitrogen atom, which comprises

a) reaction of a compound of formula II,

$R_1CHO$ II

with compounds of formulae III and IV,

$R_2OOCCH=C(R_7)NH_2$ III

$R_3OOCCH_2COR_8$ IV

in which formulae $R_1$, $R_2$, $R_3$, $R_7$ and $R_8$ are as defined above,

b) reaction of a compound of formula V,

$R_1CH=C(COOR_3)COR_8$ V

in which $R_1$, $R_3$ and $R_8$ are as defined above, with a compound of formula III,

c) production of a compound of formula I in which Y and Z together form a bond by dehydration of a compound of formula VII,

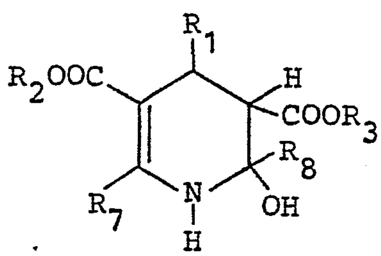

VII

in which $R_1$, $R_2$, $R_3$, $R_7$ and $R_8$ are as defined above,

d) production of a compound of formula I in which m is 1 or p is 1 or 2 by selective oxidation of a corresponding compound of formula I in which m is 0, or p is 0 or 1 respectively,

e) production of a compound of formula I in which one of $R_7$ and $R_8$ is $-CONR_{10}R_{11}$ by reaction of an acid halide, imidazole or a mixed anhydride of a corresponding compound of formula I in which one of $R_7$ and $R_8$ is -COOH with a compound $HNR_{10}R_{11}$ in which $R_{10}$ and

$R_{11}$ are as defined above, or, when the group $-NR_{10}R_{11}$ in the product represents an imidazole, reacting the free carboxylic acid of formula I with N,N'-carbonyldiimidazole,

f) production of a compound of formula I in which one of $R_7$ and $R_8$ is $-CSNH_2$ by reaction of a corresponding compound of formula I in which one of $R_7$ and $R_8$ is -CN with hydrogen sulphide,

g) isomerising a 3,4-dihydropyridine to a corresponding compound of formula I,

h) production of a compound of formula I in which one of $R_7$ and $R_8$ is $-C(=NH)SR_9$ by reaction of a corresponding compound of formula I in which one of $R_7$ and $R_8$ is $-CSNH_2$ with a compound $R_9$-hal, in which $R_9$ is as defined above and hal is a halogen atom,

i) reaction of a compound of formula IV with ammonia and a compound of formula VI,

$$R_1CH{=}C(COOR_2)COR_7 \qquad VI$$

or reaction of a compound of formula V with ammonia and a compound of formula VII,

$$R_2OOCCH_2COR_7 \qquad VII$$

or reaction of compounds of formulae II, IV and VII with ammonia,

in which formulae $R_1$, $R_2$, $R_3$, $R_7$ and $R_8$ are as defined above,

j) production of a compound of formula I in which Y and Z together form a bond and one or both of $R_7$ and $R_8$ is $-CHF_2$ or $-CH_2F$ by reaction of a corresponding compound of formula I in which Y and Z together form a bond and one or both of $R_7$ and $R_8$ is -CHO or $-CH_2L$, where L is -OH or a good leaving group, respectively with a fluorinating agent,

k) production of a compound of formula I in which one of $R_7$ and $R_8$ is -CHO by selective hydrolysis of a corresponding compound of formula I in which one of $R_7$ and $R_8$ is $-CH(OR_9)_2$,

l) production of a compound of formula I in which one of $R_7$ and $R_8$ is $-CH_2OH$ by selective reduction of a corresponding compound of formula I in which one of $R_7$ and $R_8$ is -CHO,

m) production of a compound of formula I in which one of $R_7$ and $R_8$ is -CN by elimination of ROH from a corresponding compound of formula I in which one of $R_7$ and $R_8$ is -CH=NOR, and -OR is a good leaving group,

n) production of a compound of formula I in which at least one of $R_2$ and $R_3$ is hydrogen by reductive cleavage or hydrolysis of a corresponding compound of formula I in which at least one of $R_2$ and $R_3$ is other than hydrogen,

o) production of a compound of formula I in which at

least one of $R_2$ and $R_3$ is other than hydrogen by esterification or transesterification of a corresponding compound of formula I in which at least one of $R_2$ and $R_3$ is hydrogen or is a group $R_2$ or $R_3$ other than that desired in the end product, or

p) production of an optical isomer of a compound of formula I by resolution of a mixture of optical isomers of the compound,

and where desired or necessary converting the resulting compound of formula I to a pharmaceutically acceptable acid addition salt thereof or vice versa.

2. A process according to Claim 1, wherein

$R_1$ is nitrophenyl; (trifluoromethyl)phenyl; mono- or poly-fluorophenyl; mono- or poly-chlorophenyl; chloro- and/or fluoro-(trifluoromethyl)phenyl; (alkylthio)pyridyl; alkyl- and/or chloro- and/or alkoxy-nitrophenyl; mixed chloro- and fluoro-phenyl; mono- or poly- alkoxy-phenyl; alkylphenyl; (alkylthio)phenyl; (alkylsulphonyl)phenyl, or 4-benzofurazanyl,

$R_2$ and $R_3$ are selected from alkyl C1 to 4; 2-alkoxy C1 to 3 - ethyl; 2-phenoxy- ethyl; cycloalkyl C4 to 6 optionally substituted by methyl; an oxetanyl, azetidinyl, piperidinyl or tetrahydropyranyl ring optionally substituted by phenylmethyl or diphenylmethyl; alkyl C1 to 4 - (phenylmethyl)aminoethyl; cyano- or alkyl

C1 to 4 - thio- alkyl C1 to 4; phenyl alkyl C1 to 4 or $-CH(C_6H_5)CCl_3$, .

$R_7$ is methyl, and

$R_8$ is chloro- or fluoro- alkyl C1 or 2, $-CSNH_2$, -CON(alkyl C 1 to 4)$_2$, -COmorpholino, -COimidazolyl, -C(=NH)S-alkyl C1 to 4, -S-alkyl C1 to 4, -S(O)-alkyl C1 to 4, or phenyl substituted by one or two chlorine, nitro, methoxy or methyl groups.

3. A process according to Claim 1, wherein $R_1$ is phenyl carrying a 2-nitro or a 2-$CF_3$ group or at least two substituents selected from chloro, fluoro, alkyl C1 to 6, $-CF_3$ and nitro; $R_2$ is alkyl C1 to 6, or is oxetan -3-yl, $R_3$ is alkyl C1 to 6, $R_7$ is alkyl C1 to 6, $R_8$ is fluoromethyl, and Y and Z together form a bond.

4. A process according to Claim 1, wherein $R_1$ is phenyl carrying at least two substituents selected from chloro, fluoro, $-CF_3$, methyl and nitro, $R_3$ and $R_7$ are both methyl, $R_8$ is $-CH_2F$, $R_2$ is isopropyl or cyclopentyl and Y and Z together form a bond.

5. A process according to Claim 1, wherein $R_1$ represents benzofurazanyl, pyridyl or phenyl, the pyridyl or phenyl being substituted by one or more of the groups halogen, nitro, trihalomethyl or $-SR_9$; $R_2$ and $R_3$ each represent alkyl C1 to 6, $-(CH_2)_n R_4$, $-(CH_2)_nCN$, $-CH(C_6H_5)CCl_3$ or $-(CH_2)_n$

$NR_5R_6$; Y and Z together form a bond; one of $R_7$ and $R_8$ represents alkyl C1 to 6 and the other represents $-CONR_{10}R_{11}$; $-CSNH_2$; $-C(=NH)SR_9$; $-S(O)_mR_9$; phenyl substituted by one or more of alkyl C1 to 6, halogen, alkoxy C1 to 6 or nitro; or alkyl C1 to 6 substituted by halogen; $R_4$ and $R_9$ are each alkyl C1 to 6; $R_{10}$ and $R_{11}$ each represent hydrogen or alkyl C1 to 6, n is 2, 3 or 4 and provisos i) and ii) apply.

6. A process according to Claim 1, wherein the compound of formula I is 3-Methyl 5-(1-methylethyl) 4-(3-chloro-6-fluoro-2-(trifluoromethyl)phenyl)-2-(fluoromethyl)-1,4-dihydro-6-methyl-3,5-pyridinedicarboxylate.